# EUROPEAN PATENT SPECIFICATION

(11) **EP 2 933 280 B1**
(45) Date of publication and mention of the grant of the patent: **12.10.2016**
(21) Application number: 14001428.3
(22) Date of filing: 18.04.2014
(51) Int. Cl.: C08F 283/06, C08F 220/18, C08F 220/06

(54) **Rheology modifier polymer**
Rheologiemodifikatorpolymer
Polymère modificateur de rhéologie

(43) Date of publication of application: 21.10.2015
(73) Proprietor: Rhodia Opérations, 93306 Aubervilliers (FR)
(72) Inventor: Adamy, Monique, 92600 Asnieres sur Seine (FR); Gonzales, Inigo, 60300 Mont l'Eveque (FR)
(74) Representative: Cardon, Flavie

(56) References cited:
- EP-A2- 1 488 774
- WO-A1-2011/100071
- US-A- 5 023 001

## Description

The present invention relates to new thickening emulsions comprising copolymers suitable for use in aqueous systems. These emulsions are more particularly useful for thickening personal care or cosmetic formulations in acidic conditions.

Rheology modifiers, also referred to as thickeners or viscosifiers, are ubiquitous in surfactant containing personal care cleansing formulations. Rheological properties (e.g.,viscosity and flow characteristics, foamability, spreadability, and the like), aesthetic properties (e.g., clarity, sensory effects, and the like), mildness (dermal and ocular irritation mitigation), and the ability to suspend and stabilize soluble and insoluble components within a surfactant based formulation are often modified by the addition of a thickener. Among products contributing to the rheologic behaviour of aqueous systems, in particular in the personal care field, polysaccharides and polymers based on acrylic acid also named "Carbomer" or copolymers based on acrylic acid and acrylic acid esters may be cited.

Thus, polysaccharides such as xanthan gums, carrageenan gums, succinoglycan gums etc. are well-known to provide high thickening efficiency combined with good suspension properties in a wide range of pH, including acidic pH conditions. However, they do not offer a wide tolerance to common surfactant systems and the "jelly", undesirable texture obtained with such biopolymers limits their use in cleansing products. In addition, dispersion and hydration of polysaccharides gums in water is a cumbersome task for a formulator.

Other polymer thickeners in powder form are crosslinked polymers based on acrylic acid or copolymers based on acrylic acid and acrylic acid esters. One may mention for example the polymers marketed by the company Noveon under the brand names CarbopolTM and Pemulen TM. Often, these thickeners are introduced into surfactant formulations in solid form and mixed under conditions effective to dissolve the thickener into the liquid surfactant composition in order to effect a viscosity enhancement. Frequently, the mixing must be conducted at elevated temperatures (hot processing) in order to promote the dissolution of the solid thickener and obtain the desired viscosity improvement. Additionally, solid thickeners are known to resist "wet-out" upon contact with the surface of an aqueous based system. Consequently, said solid thickeners are supplied as finely divided powders and/or must be sifted to reduce particle size, which aids in dissolution by increasing the relative surface area of the particle. Daring processing, said powders can become electrostatically charged as they are transferred in and out of containers and tend to adhere to oppositely charged surfaces including airborne dust, necessitating specialized dust extraction equipment. This means that preparation of aqueous dispersions is messy and time consuming unless special precautions and expensive equipment is employed. Formulators of compositions containing thickened surfactant constituents desire the ability to formulate their products at ambient temperatures (cold processing). Accordingly, formulators desire thickeners, which can be introduced to the liquid surfactant compositions in liquid form rather than as a solid. This provides the formulator with a greater degree of precision in introducing the thickener to the liquid surfactant composition, allows the ability to formulate products at ambient temperatures (cold processing), and better facilitates automated processing without the need for special safety and handling equipment.

One important alternative class of liquid rheology modifier commonly employed to thicken aqueous based surfactant containing formulations is the alkaliswellable or alkali-soluble emulsion (ASE) polymers and hydrophobically-modified alkali-swellable or hydrophobically-modifed alkali-soluble emulsion (HASE) polymers. ASE polymers are linear or crosslinked copolymers that are synthesized from (meth)acrylic acid and alkyl acrylates. HASE polymers, on which the present invention more particularly focuses, are linear or crosslinked copolymers that are synthesized from (meth)acrylic acid, alkyl acrylates and at least a so-called "associative" monomer.

Although these thickeners generally impart satisfactory viscosity, suspension and clarity properties in surfactant containing formulations at pH values near neutral (pH∼ 6.0), they fail to maintain at the same time all these benefits when the pH conditions vary, in particular when the surfactant containing formulations exhibit acidic pH (i.e. pH below 6).

Yet, there is nowadays a growing interest in acidic pH ranges, in particular for personal care products.

Indeed, one of the reasons why the acidic conditions are of particular interest resides in the fact that organic acids (e.g., sorbic, citric and benzoic), such as those used as preservatives in the food industry, have been increasingly looked at as the ideal replacement for traditional preservative systems, in surfactant containing formulations for safety reasons. Indeed commonly used preservatives, such as the formaldehyde donors, the halogenated compounds and the paraben compounds could have adverse effects on health.

Yet, the antimicrobial activity of the organic acids is connected to the associated or protonated species of the acid molecule. As the pH of an organic acid containing formulation increases, dissociation of the proton occurs forming acid salts. The dissociated form of the organic acids (acid salts) have no antimicrobial activity when used alone, effectively limiting the use of organic based acids to pH values below 6.

The literature has also suggested that formulating products in the natural pH range (between about 3-5) reduces the amount of preservative required in a product by enhancing preservative efficacy, stabilizes and increases the effectiveness of many cosmetic active ingredients, is beneficial to the repair and maintenance of skin barrier tissue, and supports the natural skin flora to the exclusion of over-colonization by deleterious microorganisms (US 2013/0115185).

Patent applications US 2013/183361, US 2013/0115185 and WO 2012/006402 disclose multi-staged acrylic based core-shell polymers comprising a linear core polymer and at least one other shell comprising an acrylic based crosslinked polymer stage. The core-shell polymers are said to provide desirable rheological, clarity and aesthetic properties in aqueous surfactant containing compositions, particularly at low pH. However, this technology appears to be quite overly sensitive to pH conditions since large variations of yield value (related to suspension properties), viscosity and clarity are reported depending on the pH.

Accordingly, as the industry desires new thickened surfactant based products that are formulated in the acidic pH range, there is a developing need for a rheology modifier that, especially when used in combination with a surfactant system, provides a high clarity formulation and/or formulation with a transparent visual appearance under acidic pH conditions, while maintaining a good viscosity/rheology profile, suspension (yield value), and enhanced aesthetics.

It has now been discovered a new method for manufacturing rheology modifiers, specifically HASE copolymer emulsions, that may satisfy the above mentioned need over a wide range of pH, including acidic pH conditions.

Entirely surprisingly and advantageously, the new direct emulsions of a copolymer according to the present invention, as illustrated by the examples, impart good clarity properties in the aqueous formulation in which they are used, even formulated at acidic pH ranges, while maintaining desirable rheology properties of the formulation.

Thus, the present invention relates, according to the first of its aspects, to a method for making a direct emulsion of a copolymer in water, characterized in that it comprises the polymerization of at least, expressed as a percentage by weight of each of the monomers based upon the total weight of the monomers:
a) 10 to 80% by weight of methacrylic acid and, optionally, of acrylic acid;
b) 15 to 80% by weight of at least one non-ionic vinyl monomer;
c) 0.05 to 9.5% by weight of 2-acrylamido-2-methylpropane sulfonic acid or a salt thereof;
d) 0.5 to 30% by weight of at least one monomer containing at least one hydrophobic group; and
e) 0.01 to 5% by weight of at least one crosslinking monomer;
said polymerization being carried out in the presence of at least one surfactant.

As used herein, the expression "direct emulsion of a copolymer in water" designates a stable and homogeneous dispersion of polymer particles in water.

As used herein, the term "rheology" refers to the study of the flow and deformation behavior materials and the term "rheological properties" as used herein in reference to a material or composition means the flow and deformation properties of such material or composition, including viscosity, increase or decrease in viscosity in response to shear stress or time, flow characteristics, gel properties such as stiffness, resilience, flowability, foam properties, such as foam stability, foam density, ability to hold a peak, and aerosol properties such as ability to form aerosol droplets when dispensed from propellant-based or mechanical pump-type aerosol dispensers. The term "aesthetic properties" as used herein in reference to a material or composition means the visual and tactile psychosensory properties, such as color, clarity, smoothness, tack, lubricity, texture, of such material or composition.

As used herein, the terminology "(Cₓ-C_{y})" in reference to an organic group, wherein x and y are each integers, indicates that the group may contain from x carbon atoms to y carbon atoms per group.

According to another of its aspects, the present invention relates to a direct emulsion of a copolymer in water, wherein the copolymer is polymerized from a monomer mixture comprising at least, expressed as a percentage by weight of each of the monomers based upon the total weight of monomers:
(a) 10 to 80% by weight of methacrylic acid and, optionally, of acrylic acid;
(b) 15 to 80% by weight of at least one non-ionic vinyl monomer;
(c) 0.05 to 9.5% by weight of 2-acrylamido-2-methylpropane sulfonic acid or a salt thereof;
(d) 0.5 to 30% by weight of at least one monomer containing at least one hydrophobic group; and
(e) 0.01 to 5% by weight of at least one crosslinking monomer;
wherein said emulsion contains at least one surfactant.

In the continuation, said direct emulsion of a copolymer in water according to the invention is denoted by "copolymer emulsion of the invention" or more simply "the copolymer emulsion".

In the rest of the text, the abbreviation "MA" refers to methacrylic acid, "AA" to acrylic acid and "AMPS" to 2-acrylamido-2-methylpropane sulfonic acid.

According to another of its aspects, the present invention relates to a method for thickening an aqueous composition comprising a copolymer emulsion according to the invention comprising adding to said aqueous composition a pH adjusting agent selected from an acidic material, an alkaline material and mixtures thereof.

The present invention also relates, according to still another of its aspects, to an aqueous composition comprising a copolymer emulsion according to the invention.

The present invention more particularly encompasses a composition having a pH value equal to or lower than 6, in particular equal to or lower than 5.5, more particularly ranging from 2 to 5, in particular ranging from 3 to 5.

The copolymer emulsion of the invention can thus be useful for thickening a wide variety of aqueous compositions, in particular surfactant containing formulations, that are advantageously formulated to a low pH, such as personal care, health care, home care, and institutional and industrial care compositions.

The term "personal care composition" as used herein means compositions, including but not limited to cosmetics, toiletries, cosmeceuticals, beauty aids, personal hygiene and cleansing compositions for application to the body, including the skin, hair, scalp, and nails, of humans and animals. The term "health care compositions" as used herein means compositions including but not limited to pharmaceuticals, pharmacosmetics, oral (mouth and teeth) care compositions, such as oral suspensions, mouthwashes, toothpastes, and the like, and over-the-counter compositions for external application to the body, including the skin, scalp, nails, and mucous membranes of humans and animals, for ameliorating a health-related or medical condition, or for generally maintaining hygiene or well-being. The term "home care compositions" as used herein means compositions including, but not limited to, compositions for use in a domestic household for surface cleaning or maintaining sanitary conditions, such as in the kitchen and bathroom, and laundry products for fabric care and cleaning, and the like. The term "institutional and industrial care compositions" as used herein means compositions, including but not limited to, cleaning compositions, for use in surface cleaning or maintaining sanitary conditions in institutional and industrial environments, and compositions for treating textiles.

Other features, advantages and modes of application of the copolymer emulsion according to the invention will emerge more clearly on reading the following description and examples.

In the remainder of the text, the expressions "between ... and ...", "ranging from ... to ..." and "varying from ... to ..." are equivalent and are understood to mean that the limits are included, unless otherwise mentioned.

### COPOLYMER EMULSION OF THE INVENTION

As specified previously, the copolymer emulsion according to the invention is prepared by polymerizing at least, expressed as a percentage by weight of each of the monomers based upon the total weight of the monomers:
a) 10 to 80% by weight of MA and, optionally, of AA;
b) 15 to 80% by weight of at least one non-ionic vinyl monomer;
c) 0.05 to 9.5% by weight of AMPS or a salt thereof;
d) 0.5 to 30% by weight of at least one monomer containing at least one hydrophobic group; and
e) 0.01 to 5% by weight of at least one crosslinking monomer;
said polymerization being carried out in the presence of at least one surfactant.

According to a particularly preferred embodiment, the copolymer does not contain any other monomer units. In other words, the total of a), b), c), d) and e) weight contents is preferably equal to 100%.The copolymer emulsion of the invention can be conveniently prepared from the above-mentioned monomers by known aqueous emulsion polymerization techniques.

In one embodiment, the polymerization is conducted in the presence of one or more free-radical producing initiators selected from peroxygen compounds.

Useful peroxygen compounds include inorganic persulfate compounds such as ammonium persulfate, potassium persulfate, sodium persulfate, peroxides such as hydrogen peroxide, organic hydroperoxides, for example, curnene hydroperoxide, and t-butyl hydroperoxide, organic peroxides, for example, benzoyl peroxide, acetyl peroxide, lauroyl peroxide, peracetic acid, and perbenzoic acid (sometimes activated by a water-soluble reducing agent such as ferrous compound or sodium bisulfite), and other free-radical producing materials or techniques such as 2,2'-azobisisobutyronitrile and high energy radiation sources.

The emulsion polymerization may, optionally, be conducted in the presence, in an amount up to about 10 parts per 100 parts of polymerizable monomers, of one or more chain transfer agents.

Representative chain transfer agents are carbon tetrachloride, bromoform, bromotrichloromethane, and long-chain alkyl mercaptans and thioesters, such as n-dodecyl mercaptan, t-dodecyl mercaptan, octyl mercaptan, tetradecyl mercaptan, hexadecyl mercaptan, butyl thioglycolate, isooctyl thioglycolate, and dodecyl thioglycolate.

Optionally, other ingredients well known in the emulsion polymerization art may be included, such as chelating agents, buffering agents, inorganic salts and pH adjusting agents.

In one embodiment, the polymerization is carried out at a temperature of from about 20°C to 100°C, more typically from about 50°C to about 90°C or between about 60°C and about 90°C, and even more typically from about 60°C to about 80°C, but higher or lower temperatures may be used.

The polymerization can be conducted batchwise, stepwise, or continuously with batch and/or continuous addition of the monomers, in a conventional manner.

The monomers can be copolymerized in such proportions, and the resulting polymers can be physically blended, to give products with the desired balance of properties for specific applications.

The polymeric products according to the present invention prepared by emulsion polymerization at an acid pH are in the form of stable aqueous colloidal dispersions containing the polymer dispersed as discrete particles having average particle diameters of about 400 to about 3000 Å and preferably about 500 to about 1750 Å, as measured by light scattering. Dispersions containing polymer particles smaller than about 400 Å are difficult to stabilize, while particles larger than about 3000 Å reduce the ease of dispersion in the aqueous products to be thickened. In one embodiment, the polymer composition of the present invention is in the form of an aqueous polymer dispersion, typically having a solids content including the polymer and surfactant(s), based on the total weight of the polymer dispersion, of up to about 60 wt% and, more typically about 20 to about 50 wit%.

Alternatively, polymers according to the present invention can be made using known solution polymerization techniques, wherein the reactant monomers and initiator are dissolved in an appropriate solvent such as toluene, xylene, tetrahydrofuran, or mixtures thereof. Polymerization can be accomplished within the time necessary at a given reaction temperature, e.g., from about 60°C to about 80°C for from about 2 to about 24 hours. The polymer product can be isolated through normal separation techniques, including solvent stripping.

### MA (and optionally AA) monomer

According to a particular embodiment, the copolymer is prepared by the polymerisation of at least 10.5 to 80% by weight of both MA and AA.

According to a particular embodiment, the total weight amount of methacrylic acid and, optionally, of acrylic acid, for example the total weight amount of methacrylic acid and of acrylic acid, ranges from 20% to 50%, in particular from 25% to 40%, based upon the total weight of the monomers forming the copolymer.

According to an embodiment variant, when said copolymer is polymerized from both AA and MA units, the weight ratio of MA to AA may range from 0.1 to 50, in particular from 0.2 to 30 and more particularly from 1 to 20.

### Non-ionic vinyl monomer

More particularly, non-ionic vinyl monomers suitable for use in the present invention are copolymerizable, non-ionic, ethylenically unsaturated monomers, which are well known in the art. Preferred non-ionic vinyl monomers are compounds having either of the following formulas (I) or (II):

(I) CH₂=C(X)Z,

(II) CH₂=CH-OC(O)R;

wherein, in each of formulas (I) and (II), X is H or methyl; and Z is -C(O)OR¹, -C(O)NH₂, -C(O)NBR¹, -C(O)N(R¹)₂, -C₆H₄R¹, -C₆H₄OR¹ ,-C₆H₄Cl, -CN,-NHC(O)CH₃, -NHC(O)H, N-(2-pyrrolidonyl), N-caprolactamyl, -C(O)NHC(CH₃)₃,-C(O)NHGH₂CH₂-N-ethyleneurea, -SiR₃, -C(O)O(CH₂)ₓSiR₃, -C(O)NH(CH₂)ₓSiR₃, or-(CH₂)ₓSiR₃; x is an integer in the range of 1 to 6; each R is independently C₁-C₁₈ alkyl; each R¹ is independently C₁-C₃₀ alkyl, hydroxy-substituted C₂-C₃₀ alkyl, or halogen-substituted C₁-C₃₀ alkyl.

Non-limiting examples of suitable water-insoluble non-ionic vinyl monomers include C₁-C₃₀ alkyl (meth)acrylates; C₁-C₃₀ alkyl (meth)acrylamides; styrene; substituted styrenes, such as vinyl toluene (e.g. , 2-methyl styrene), butyl styrene, isopropyl styrene, p-chloro styrene, and the like; vinyl esters, such as vinyl acetate, vinyl butyrate, vinyl caprolate, vinyl pivalate, vinyl neodecanoate, and the like; unsaturated nitrites, such as methacrylonitrile, acrylonitrile, and the like; and unsaturated silanes, such as trimethylvinylsilane, dimethylethylvinylsilane, allyldimethylphenylsilane, allytrimethylsilane, 3-acrylamidopropyltrimethylsilane, 3-trimethylsilylpropyl methacrylate, and the like.

Non-limiting examples of suitable water-soluble nonionic vinyl monomers are C₂-C₆ hydroxyalkyl (meth)acrylates; glycerol mono(meth)acrylate; tris(hydroxymethyl)ethane mono(meth)acrylate; pentaerythritol mono(meth)acrylate; N-hydroxymethyl (meth)acrylamide; 2-hydroxyethyl (meth)acrylamide; 3-hydroxypropyl (meth)acrylamide; (meth)acrylamide; N-vinyl caprolactam; N-vinyl pyrrolidone; methacrylamidoethyl-N-ethyleneurea (e.g. , CH₂=C(CH₃)C(O)NHCH₂CH₂-N-ethyleneurea), C₁-C₄ alkoxy-substituted (meth)acrylates and (meth)acrylamides, such as methoxyethyl (meth)acrylate, 2-(2-ethoxyethoxy)ethyl (meth)acrylate, and the like; and combinations thereof.

Particularly preferred nonionic vinyl monomers include esters of acrylic acid and/or of methacrylic acid (also noted "esters of (meth)acrylic acid), methacrylamidoethyl-N-ethylene urea, and combinations thereof.

In a particularly preferred embodiment, the non-ionic vinyl monomer is an ester of (meth)acrylic acid, for example chosen from C₁-C₁₈-alkyl esters of acrylic acid and/or of methacrylic acid, in particular chosen from ethyl acrylate, butyl acrylate, and methyl methacrylate.

Preferably, the non-ionic vinyl monomer is ethyl acrylate.

In a particular embodiment, the weight amount of non-ionic vinyl monomer(s) is greater than or equal to 30%, for example ranges from 30% to 80%, for example is greater than or equal to 40%, for example is greater than or equal to 50%, for example ranges from 50% to 75%, based upon the total weight of the monomers forming the copolymer.

### AMPS monomer

More particularly, the 2-acrylamido-2-methylpropane sulfonic acid monomer (AMPS) or salts thereof represent from from 0.1% to 7%, in particular from 0.5% to 5% and more particularly from 1% to 3%, based upon the total weight of the monomers forming the copolymer.

Suitable salts include alkali metal salts, such as sodium, potassium and lithium salts; alkaline earth metal salts, such as calcium and magnesium salts; ammonium salts; and alkyl-subsituted ammonium salts, such as salts of 2-amino-2-methyl-1-propanol (AMP), ethanolamine, diethanolamine, triethanolamine and triethanolamine. Preferred salts of AMPS arc sodium and ammonium salts of AMPS.

### Monomer containing at least one hydrophobic group

More particularly, the monomer(s) containing at least one hydrophobic group, referred below as "associative monomer", is/are chosen from the monomers of general structure (III): 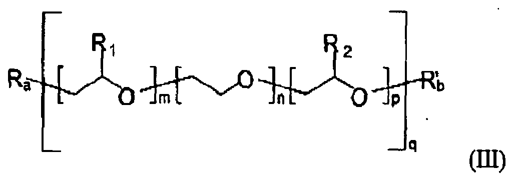 wherein:
- m, n, p and q are integers and m, n, p are inferior to 150, q is superior to 0 and at least one integer from m, n and p is non-zero,
- Ra has a polymerizable vinylic function;
- R₁ and R₂ are indentical or different and represent hydrogen atoms or alkyl groups,
- R'b is a hydrophobic group containing from 6 to at most 36 carbon atoms.

In a particular embodiment, the associative monomer may be chosen from monomers of general structure (IV): 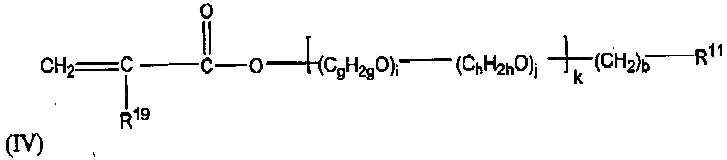 wherein:
R¹¹ is bicyclo[d.e.f]heptyl or bicyclo[d.e.f]heptenyl wherein d is 2,3, or 4, e is 1 or 2, f is 0 or 1, and the sum of d + e + f = 5, and which may, optionally, be substituted on one or more of the ring carbon atoms by one or more (C₁-C₆)alkyl groups, and
b is an integer of from 1 to 6,
g and h are independently integers of from 2 to 5, more typically 2 or 3,
each i is independently an integer of from 1 to 80, more typically from 1 to about 50,
each j is independently an integer of from 0 to 80, more typically from 1 to about 50 and
k is an integer of from 1 to 50, provided that the product obtained by multiplying the integer k times the sum of i+j is from 2 to 100.

According to one embodiement, the associative monomer may be chosen from monomers of general structure (IV) wherein R₁₉ is H or methyl, b is an integer of from 1 to 6, g = 2, h = 3, i is an integer of from 1 to 50, more typically 10 to 40, and even more typically from 15 to about 30 j is an integer of from 1 to 30, more typically from 2 to 20, and even more typically from about 2 to about 10, and k = 1.

Preferably, the associative monomer may comprises one or more compounds according to structure (IV'): 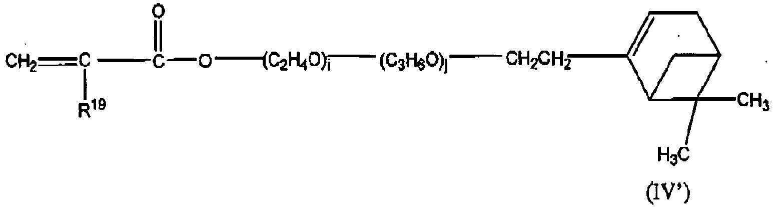 wherein i, j, and R¹⁹ are each as described above, and, more typically, i is an integer of from 10 to 40, and even more typically from 15 to about 30, and still more typically form 20 to 30, and j is an integer of from 1 to 20, and even more typically from about 2 to about 10, and still more typically from 3 to 8.

In another particular embodiment, the associative monomer may be chosen from monomers of general structure (V): 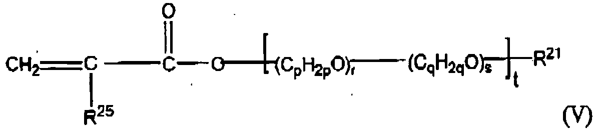 wherein:
R²¹ is linear or branched (C₅-C₅₀)alkyl, hydroxyalkyl, alkoxyalkyl, aryl, or aralkyl,
R²⁵ is methyl or ethyl
p and q are independently integers of from 2 to 5,
each r is independently an integer of from 1 to 80,
each s is independently an integer of from 0 to 80, and
t is an integer of from 1 to 50, provided that the product obtained by multiplying the integer t times the sum of r+s is from 2 to 100.
In one embodiment, the associative monomer may be chosen from monomers of general structure (V) wherein R²¹ is linear (C₁₆-C₂₂)alkyl.
In one embodiment, the associative monomer may be chosen from monomers of general structure (V) wherein p = 2, s =0, and t =1.

In one embodiment, the associative monomer may be chosen from monomers of general structure (V) wherein R²¹ is linear (C₁₆-C₂₂)alkyl, R²⁵ is methyl or ethyl, p = 2, s =0, and t

According to an embodiment, the copolymer of the invention is polymerized from at least one first associative monomer of general structure (IV), preferably of structure (IV') as specified above, and from at least one second associative monomer of general structure (V), in particular a mixture of associative monomers of formula (V) as specified above.

According to this particular embodiment, the weight ratio of first associative monomer(s) to second associative monomer(s) preferably ranges from 0.05 to 10, in particular from 0.1 to 5 and more particularly from 0.2 to 1.0.

According to a particular embodiment, the associative monomers represent from 1 to 25% by weight, preferably from 5 to 20% by weight, based upon the total weight of the monomers forming the copolymer of the invention.

### Crosslinking monomers

Suitable crosslinking monomers are compounds having more than one reactive functional group, such as for example, more than one site of unsaturation, typically ethylenic unsaturation, per molecule, that are copolymerizable with the reactive functional groups of the other monomers of mixture under the polymerization reaction conditions used. Suitable polyunsaturated crosslinkers are well known in the art. Monounsaturated compounds that further comprise a second reactive functional group that is capable of causing a formed copolymer to be crosslinked before, during, or after polymerization has taken place can also be utilized. Other useful crosslinking monomers include polyfunctional monomers containing multiple reactive groups, such as epoxide groups, isocyanate groups, and hydrolyzable silane groups. Various polyunsaturated compounds can be utilized to generate either a partially or substantially cross-linked three dimensional network. Suitable polyunsaturated crosslinker monomers include, for example, polyunsaturated aromatic monomers, such as divinylbenzene, divinyl naphthalene, and trivinylbenzene, polyunsaturated alicyclic monomers, such as 1,2,4-trivinylcyclohexane, di-functional esters of phthalic acid, such as diallyl phthalate, polyunsaturated aliphatic monomers, such as dienes, trienes, and tetraenes, including isoprene, butadiene, 1,5-hexadiene, 1,5,9-decatriene, 1,9-decadiene, 1,5-heptadiene, polyalkenyl ethers, such as triallyl pentaerythritol, diallyl pentaerythritol, diallyl sucrose, octaallyl sucrose, and trimethylolpropane diallyl ether, polyunsaturated esters of polyalcohols or polyacids, such as 1,6-hexanediol di(meth)acrylate, tetramethylene tri(meth)acrylate, allyl acrylate, diallyl itaconate, diallyl fumarate, diallyl maleate, trimethylolpropane tri(meth)acrylate, trimethylolpropane di(meth)acrylate, poly(alkyleneoxy) glycol di(meth)acrylates, and polyethylene glycol di(meth)acrylate, alkylene bisacrylamides, such as methylenebisacrylamide and propylene bisacrylamide, hydroxy and carboxy derivatives of methylene bis-acrylamide, such as N,N'-bismethylol methylene bisacrylamide, polyalkyleneglycol di(meth)acrylates, such as ethyleneglycol di(meth)acrylate, diethyleneglycol di(meth)acrylate, and triethyleneglycol di(meth)acrylate, polyunsaturated silanes, such as dimethyldivinylsilane, methyltrivinylsilane, allyldimethylvinylsilane, diallydimethylsilane and tetravinylsilane, polyunsaturated stannanes, such as tetraallyl tin, diallyldimethyl tin. In one embodiment, the copolymer of the present invention comprises crosslinks derived from one or more crosslinker monomers having more than one (meth)acrylic group per molecule, such as, for example, allyl methacrylate, ethylene glycol dimethacrylate, butylene glycol dimethacrylate, diallyl pentaerythritol, methylenebisacrylamide, pentaerythritol di-, tri- and tetra-acrylates, divinyl benzene, poly(alkyleneoxy)glycol di(meth)acrylates, such as polyethylene glycol diacrylates, bisphenol A diacrylates, butanediol dimethacrylate, 2,2-dimethylpropanediol dimethacrylate, ethylene glycol dimethacrylate, phenylene diacrylate, or a mixture thereof.

According to a particular embodiment, the crosslinking monomer(s) is/are used in an amount ranging from 0.01% to 5% by weight relative to the total weight of the monomers, in particular lower than 3%, more particularly ranging from 0.05% to 1% by weight, relative to the total weight of the monomers.

### Surfactant

According to another essential feature of the invention, the polymerization is carried out in the presence of at least one surfactant

Surfactants for emulsion polymerizations include anionic, nonionic, amphoteric, and cationic surfactants, as well as mixtures thereof. Most commonly, anionic and nonionic surfactants can be utilized as well as mixtures thereof.

Suitable anionic surfactants for emulsion polymerizations are well known in the art and include, but are not limited to, sodium lauryl sulfate, sodium dodecyl benzene sulfonate, sodium (C₆-C₁₆) alkyl phenoxy benzene sulfonate, disodium (C₆-C₁₆) alkyl phenoxy benzene sulfonate, disodium (C₅-C₁₆) di-alkyl phenoxy benzene sulfonate, disodium laureth-3 sulfosuccinate, sodium dioctyl sulfosuccinate, sodium di-sec-butyl naphthalene sulfonate, disodium dodecyl diphenyl ether sulfonate, disodium n-octadecyl sulfosuccinate, phosphate esters of branched alcohol ethoxylates, sodium or ammonium salts of sulphated (C₆-C₁₆) alcohol ethoxylate and the like.

Nonionic surfactants suitable for emulsion polymerizations are well known in the polymer art, and include, without limitation, linear or branched C₈-C₃₀ fatty alcohol ethoxylates, such as capryl alcohol ethoxylate, lauryl alcohol ethoxylate, myristyl alcohol ethoxylate, cetyl alcohol ethoxylate, stearyl alcohol ethoxylate, cetearyl alcohol ethoxylate, sterol ethoxylate, oleyl alcohol ethoxylate, and, behenyl alcohol ethoxylate; alkylphenol alkoxylates, such as octylphenol ethoxylates; and polyoxyethylene polyoxypropylene block copolymers, and the like. Additional fatty alcohol ethoxylates suitable as non-ionic surfactants are described below. Other useful nonionic surfactants include C₈-C₂₂ fatty acid esters of polyoxyethylene glycol, ethoxylated mono- and diglycerides, sorbitan esters and ethoxylated sorbitan esters, C₈-C₂₂ fatty acid glycol esters, block copolymers of ethylene oxide and propylene oxide, and combinations thereof. The number of ethylene oxide units in each of the foregoing ethoxylates can range from 2 and above in one aspect, and from 2 to 150 in another aspect.

In a particularly preferred embodiment, the surfactant is chosen from anionic surfactants selected from sodium dodecyl benzene sulfonate, sodium dodecyl butylnaphthalene sulfonate, sodium lauryl sulfate, ammonium lauryl sulfate, ammonium laureth sulfate, disodium dodecyl diphenyl ether disulfonate, disodium n-octadecyl sulfosuccinamate, sodium or ammonium salts of sulphated (C₆-C₁₆) alcohol ethoxylate and sodium dioctyl sulfosuccinate, and from nonionic surfactants, in particular selected from fatty alcohols, alkoxylated fatty alcohols, and allylpolyglucosides.

The surfactant(s) is/are used in an active content greater than 0.1% by weight relative to the total active weight of monomers, in particular ranging from 0.2 to 5% and more particularly ranging from 0.3 to 3% by weight, relative to the total active weight of the monomers.

In a particular embodiment, the final copolymer of the invention exhibits an average molar mass by weight of between 20,000 and 1,000,000 g/mol.

According to a particular embodiment, the copolymer emulsion of the invention has a solids content of between 10 and 50% by dry weight of copolymer in relation to its total weight, in particular between 15 and 40%.

### COMPOSITIONS AND APPLICATIONS

### Aqueous compositions

As mentioned previously, the copolymer emulsions of the invention are particularly useful for thickening a wide variety of aqueous compositions, in particular surfactant containing compositions, such as personal care, home care, health care, and institutional and industrial care compositions.

According to one of its aspects, the present invention thus encompasses a method for thickening an aqueous composition comprising an emulsion of a copolymer according to the invention, comprising adding to said aqueous composition a pH adjusting agent selected from an acidic material, an alkaline material and mixtures thereof.

The copolymer emulsions of the invention are generally supplied in their acidic form. As it is well known for HASE type polymers, these copolymer emulsions modify the rheology of a formulation through the neutralization of the carboxy groups on the polymer with an alkaline material.

Advantageously, the composition comprising a copolymer emulsion of the invention can be acidified subsequent to neutralization to reach the desired low pH value, without affecting its rheology and clarity properties.

Thus, according to a particular embodiment, the pH of the composition comprising a copolymer emulsion of the invention is first adjusted with an alkaline pH adjusting agent to 0.5 to 4 pH units above the initial pH of the composition and subsequently the alkaline adjusted pH is reduced by adding an acidic pH adjusting agent in a sufficient amount to obtain a final pH value equal to or lower than 6.

In particular, the alkaline pH adjusting agent can be added and mixed to increase the pH of the aqueous composition in which the copolymer emulsion of the invention is formulated, to increase the pH of the composition to strictly greater than 6, in particular greater than or equal to 6.5.

Any material capable of increasing the pH of the composition is suitable. For example, the alkaline pH adjusting agent incorporated to neutralize the copolymer may be selected from sodium hydroxide, potassium hydroxide, triethanolamine, or another fatty acid amine neutralizing agent commonly used in said applications.

Subsequent to the pH adjustment with the alkaline material, an acidic material is added to reduce the pH of the composition to the desired target pH for the composition.

For example, the acidic pH adjusting agent may be selected from organic acids and inorganic acids, for example, acetic acid, citric acid, tartaric acid, alpha-hydroxy acids, beta-hydroxy acids, salicylic acid, lactic acid, glycolic acid, and natural fruit acids, or inorganic acids, for example, hydrochloric acid, nitric acid, sulfuric acid, sulfamic acid, phosphoric acid, and combinations thereof. The pH adjusting agents can be added at any stage of the preparation of the composition.

In one embodiment, the pH of the composition comprising a copolymer emulsion of the invention can be directly adjusted with an alkaline pH adjusting agent at a value equal to or lower than 6.

Alternatively, the pH of the composition comprising a copolymer emulsion of the invention can be directly adjusted with an alkaline pH adjusting agent at a value greater than 6.

In one aspect of the invention, the target final pH of the composition is equal to or lower than 5.5, more particularly ranging from 2 to 5, in particular ranging from 3 to 5.

In a particularly preferred embodiment, the copolymer emulsion of the invention is used in an amount ranging from 0.5% to 10%, in particular from 1% to 5%, by dry weight of copolymer relative to the total weight of the aqueous composition. The polymer may typically be added at any stage or at multiple stages of the preparation of composition, such as, by addition to water before addition of other ingredients, by addition to the composition among other added ingredients, or by addition after addition of any other ingredients, as the final ingredient in a series of additions and/or as a post-addition to the composition, such as, for example, as a post-addition to adjust the rheological properties of the composition.

### Surfactant

According to a particular embodiment, the aqueous composition wherein the copolymer emulsion of the invention is used may further include at least one surfactant different from the surfactant contained in said emulsion of the copolymer. The surfactant may be selected from anionic, zwitterionic or amphoteric, cationic or non-ionic surfactants, and combinations thereof.

The anionic surfactant can be any of the anionic surfactants known or previously used in the art of aqueous surfactant compositions. Suitable anionic surfactants include but are not limited to alkyl sulfates, alkyl ether sulfates, alkyl sulphonates, alkaryl sulfonates, alpha-olefin-sulphonates, alkylamide sulphonates, alkarylpolyether sulphates, alkylamidoether sulphates, alkyl monoglyceryl ether sulfates, alkyl monoglyceride sulfates, alkyl monoglyceride sulfonates, alkyl succinates, alkyl sulfosuccinates, alkyl sulfosuccinamates, alkyl ether sulphosuccinates, alkyl amidosulfosuccinates; alkyl sulphoacetates, alkyl phosphates, alkyl ether phosphates, alkyl ether carboxylates, alkyl amidoethercarboxylates, N-alkylamino acids, N-acyl amino acids, alkyl peptides, N-acyl taurates, alkyl isethionates, carboxylate salts wherein the acyl group is derived from fatty acids; and the alkali metal, alkaline earth metal, ammonium, amine, and triethanolamine salts thereof.

In one aspect, the cation moiety of the forgoing salts is selected from sodium, potassium, magnesium, ammonium, mono-, di- and triethanolamine salts, and mono-, di-, and tri-isopropylamine salts.

Examples of suitable anionic surfactants include sodium, potassium, lithium, magnesium, and ammonium salts of laureth sulfate, trideceth sulfate, myreth sulfate, C₁₂-C₁₃ pareth sulfate, C₁₂-C₁₄ pareth sulfate, and C₁₂-C₁₅ pareth sulfate, ethoxylated with 1,2, and 3 moles of ethylene oxide; sodium, potassium, lithium, magnesium, ammonium, and triethanolamine lauryl sulfate, coco sulfate, tridecyl sulfate, myristyl sulfate, cetyl sulfate, cetearyl sulfate, stearyl sulfate, oleyl sulfate, and tallow sulfate, disodium lauryl sulfosuccinate, disodium laureth sulfosuccinate, sodium cocoyl isethionate, sodium C₁₂-C₁₆ olefin sulfonate, sodium laureth-6 carboxylate, sodium methyl cocoyl taurate, sodium cocoyl glycinate, sodium lauroyl glycinate, sodium myristyl sarcocinate, sodium dodecylbenzene sulfonate, sodium cocoyl sarcosinate, sodium cocoyl glutamate, potassium myristoyl glutamate, triethanolamine monolauryl phosphate, and fatty acid soaps, including the sodium, potassium, ammonium, and triethanolamine salts of a saturated and unsaturated fatty acids containing from about 8 to about 22 carbon atoms.

Cationic surfactants are generally known and include for example, mono-cationic surfactants according to formula (XXV): 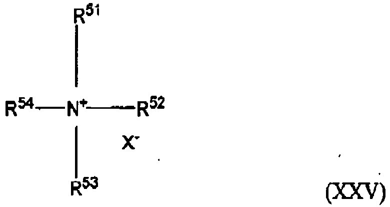 wherein:
R⁵¹, R⁵², R⁵³, and R⁵⁴ are each independently H or an organic group, provided that at least one of R⁵¹, R⁵², R⁵³, and R⁵⁴ is not hydrogen, and
X⁻ is an anion, more, chloride, bromide, methosulfate, ethosulfate, lactate, saccharinate, acetate or phosphate anion.

If one to three of R⁵¹, R⁵², R⁵³, and R⁵⁴ of the compound of structure (XXV) are each H, then the compound according to structure (XXV) is an amine salt. Suitable amine salt type cationic surfactants include polyethoxylated (2) oleyl/stearyl amine, ethoxylated tallow amine, cocoalkylamine, oleylamine, and tallow alkyl amine.

If R⁵¹, R⁵², R⁵³, and R⁵⁴ of the compound of structure XXV are each independently an organic group, then the compound of structure XXV is a quaternary ammonium compound. In one embodiment, R⁵¹, R⁵², R⁵³, and R⁵⁴ are each independent (C₈-C₂₄) branched or linear hydrocarbon groups which may comprise additional functionality such as, for example, fatty acids or derivatives thereof, including esters of fatty acids and fatty acids with alkoxylated groups, alkyl amido groups, aromatic rings, heterocyclic rings, phosphate groups, epoxy groups, and hydroxyl groups. The nitrogen atom may also be part of a heterocyclic or aromatic ring system, e.g., cataphyll morpholinium ethosulfate or steapyrium chloride. Specific examples of suitable quaternary ammonium compounds include cetyl trimethyl ammonium bromide, cetyl trimethyl ammonium chloride, myristyl trimethyl ammonium bromide, stearyl dimethyl benzyl ammonium chloride, oleyl dimethyl benzyl ammonium chloride, lauryl/myristryl trimethyl ammonium methosulfate, cetyl dimethyl (2)hydroxyethyl ammonium dihydrogen phosphate), cocotrimonium chloride, distearyldimonium chloride, stearyl octyldimonium methosulfate, isostearaminopropalkonium chloride, dihydroxypropyl PEG-5 linoleaminium chloride, PEG-2 stearmonium chloride, Quaternium 18, Quaternium 32, Quaternium 80, Quaternium 82, Quaternium 84, behentrimonium chloride, dicetyl dimonium chloride, behentrimonium methosulfate, tallow trimonium chloride, behenamidopropyl ethyl dimonium ethosulfate, distearyldimonium chloride, dicetyl dimonium chloride, stearyl octyldimonium methosulfate, dihydrogenated palmoylethyl hydroxyethylmonium methosulfate, dipalmitoylethyl hydroxyethylmonium methosulfate, dioleoylethyl hydroxyethylmonium methosulfate, hydroxypropyl bisstearyldimonium chloride, isostearyl benzylimidonium chloride, cocoyl benzyl hydroxyethyl imidazolinium chloride, cocoyl hydroxyethylimidazolinium PG-chloride phosphate, stearyl hydroxyethylimidonium chloride, and mixtures thereof.

Amphoteric surfactants are generally known. Suitable amphoteric surfactants include the alkali metal, alkaline earth metal, ammonium or substituted ammonium salts of alkyl amphodipropionates, alkyl amphoacetates, alkyl amphodiacetates, alkyl amphoglycinates, and alkyl amphopropionates, as well as alkyl iminopropionates, alkyl iminodipropionates, and alkyl amphopropylsulfonates, such as for example, cocoamphoacetate cocoamphopropionate, cocoamphodiacetate, lauroamphoacetate, lauroamphodiacetate, lauroamphodipropionate, lauroamphodiacetate, cocoamphopropylsulfonate, caproamphodiacetate, caproamphoacetate, caproamphodipropionate, and stearoannphoacetate, cocoamidopropyl hydroxysultaine, and mixtures thereof. Specific examples of suitable amphoteric surfactant include sodium lauroamphoacetate, sodium lauroamphopropionate, disodium lauroamphodiacetate, sodium cocoamphoacetate, disodium cocoamphodiacetate, and mixtures thereof.

Zwitterionic surfactants are generally known and include betaine surfactants and sultaine surfactants, such as for example decyl dimethyl betaine, undecyl dimethyl betaine, dodecyl dimethyl betaine, tridecyl dimethyl betaine, tetradecyl dimethyl betaine, coco dimethyl betaine, hexadecyl dimethyl betaine, heptadecyl dimethyl betaine, octadecyl dimethyl betaine, dodecylamidopropyl dimethyl betaine, cocoamidopropyl betaine cocoamidopropyl dimethyl betaine, oleylamidopropyl betaine, lauryl dihydroxypropyl glycinate, lauryl di(hydroxy-poly(ethoxy)) glycinate, lauryl bis-(2-hydroxy-ethyl)carboxy methyl betaine, stearyl bis-(2-bydroxy-propyl)carboxymethyl betaine, cocodimethyl sulfopropyl betaine, stearyldimethyl sulfopropyl betaine, lauryl dimethyl sulfoethyl betaine, lauryl bis-2-hydroxy-ethyl)sulfopropyl betaine, cocoamidopropyl hydroxysultaine and mixtures thereof.

Nonionic surfactants are generally known and include, for example, alkanolamides, which may optionally be alkoxylated, amine oxides, fatty alcohols, which may optionally be alkoxylated, alkoxylated alkyl phenols, fatty acid esters, and alkylglucosides, such as cocamide DEA, cocamide MEA, cocamide MIPA, PEG-5 cocamide MEA, lauramide DEA, lauramine oxide, cocamine oxide, stearamine oxide, stearamidopropylamine oxide, palmitanidopropylamine oxide, decylamine oxide, stearyl alcohol, sorbitan monolaurate, polysorbates, ethoxylated lauryl alcohols, polyethylene glycol distearates, decyl glucosides, coco glucosides, dodecyl glucosides, octadecyl polyglucosides, and mixtures thereof.

Typically the aqueous composition wherein the copolymer emulsion of the invention is used may comprise at least 5% by weight of surfactants (expressed as active material) relative to the total weight of the aqueous composition.

The copolymer emulsion of the present invention can be used in combination with conventional polymeric thickeners, such as natural gums, resins, polysaccharides, synthetic polymeric thickeners, and the like, commonly used in the art in a way that the expected effect of clarity of the aqueous system is not affected by such combination.

Of course, the compositions of the invention can contain other additives, depending on the desired end-use application.

For example, the compositions can comprise any combination of optional additives, adjuvants and benefit agents suitable for a desired personal care, home care, health care, institutional or industrial care product known in the art. The choice and amount of each optional component employed will vary with the purpose and character of the end product, and can be readily determined by one skilled in the formulation art and from the literature.

Optional additives and adjuvants include, but are not limited to insoluble materials, pharmaceutical and cosmeceutical actives, chelators, conditioners, diluents, solvents, fragrances, humectants, lubricants, solubilizers, emollients, opacifiers, colorants, anti-dandruff agents, preservatives, spreading aids, emulsifiers, sunscreens, fixative polymers, botanicals, and the like, as well as the numerous other optional components for enhancing and maintaining the properties of a desired personal care, home care, health care, and I&I care composition.In one embodiment, the additive may be a benefit agent comprising a cationic polymer and/or an amphoteric polymer. Suitable cationic polymers include synthetic polymers that comprise monomeric units derived from one or more amine- and/or quaternary ammonium-substituted monomers and natural polymers that have been derivatized to include amine- and/or quaternary ammonium-containing pendant groups, each typically having a cationic charge density of from about 0.1 to 4 meq/g. Suitable cationic polymers include, for example, copolymers of 1-vinyl-2-pyrrolidine and 1-vinyl-3-methyl-imidazolium salts (such as Polyquatemium-16), copolymers of 1-vinyl-2-pyrrolidine and dimethylaminoethyl methacrylate (such as Polyquaternium-11), cationic diallyl quaternary ammonium-containing polymers including, for example, dimethyldiallyammonium chloride homopolymers and copolymers of acrylamide and dimethyldiallylammonium chloride (such as Polyquaternium 6 and Polyquaternium 7), cationic polyacrylamides, cationic polysaccharide polymers, such as, for example, cationic cellulose derivatives, cationic starch derivatives, and cationic guar gum derivatives, such as salts of hydroxyethyl cellulose reacted with trimethyl ammonium substituted epoxide (such as Polyquaternium 10), polymeric quaternary ammonium salts of hydroxyethyl cellulose reacted with lauryl dimethyl ammonium-substituted epoxide (such as Polyquaternium 24), guar hydroxypropyltrimonium chloride, hydroxypropyl guar hydroxypropyltrimonium chloride and cationic protein derivatives, such as cocodimonium hydroxypropyl hydrolyzed wheat protein. Suitable amphoteric polymers are polymers that contain both anionic groups, such as phosphate, phosphonate, sulphate, sulphonate or carboxylic acid groups, and cationic groups, such as tertiary amino groups or quaternary ammonium groups, on the same polymer molecule. Suitable amphoteric polymers include, for example, amphoteric acrylic copolymers, such as octylacrylamide / acrylate / butylaminoethyl methacrylate copolymers, and amphoteric polysaccharide compounds obtained by grafting and polymerization of cationic pendant groups, e.g., dimethyldiallylammonium chloride groups, onto anionic polysaccharide, for example, a sodium carboxymethyl-cellulose, backbone Aqueous compositions containing the polymer of the present invention, one or more surfactants and/or non-surfactants salts, and a cationic polymer and/or amphoteric polymer exhibit an enhanced thickening efficiency compared to analogous compositions that lack the cationic polymer and/or amphoteric polymer.

### Properties

As mentioned above, the copolymer emulsion of the present invention may impart at the same time acceptable viscosity, suspension and clarity properties in aqueous formulations, especially at acidic pH values.

In particular, the copolymer emulsion of the invention may provide thickening and impart a non-zero yield strength.

In one embodiment, an aqueous composition comprising the copolymer of the present invention exhibits non-Newtonian "shear thinning" viscosity, that is, a viscosity that, within a given range of shear stress, decreases with increasing shear stress.

In one embodiment, an aqueous composition comprising the copolymer of the present invention exhibits a "yield strength", that is, a minimum shear stress required to initiate flow of the composition, and exhibits shear thinning behavior over some range of shear stress above the yield strength, such as for example, a yield strength of greater than 0 Pa, more typically of from 0.1 Pa and even more typically from 1 Pa, to 10 Pa, more typically to 6 Pa, and even more typically to 2 Pa.

Advantageously, an aqueous composition of the invention, for example a personal care composition, thus comprises an amount of copolymer effective to impart a yield strength of greater than 0 Pa to the composition, in particular greater than 0.5. Pa and more particularly greater than 1 Pa.

A non-zero yield strength is useful for suspending water insoluble particles in a composition according to the invention. Such water insoluble particles may include abrasives, pigments, oil droplets, oil beads, liposomes, capsules, gas bubbles, etc., depending on the purpose and character of the composition comprising the copolymer emulsion of the invention.

Thus, in a particular embodiment, the aqueous composition of the invention comprises an amount of copolymer effective to impart a yield strength of greater than 1 Pa to said composition, and the composition further comprises suspended particles of one or more solid, liquid or gas that are insoluble or are only partly soluble in the composition.

Advantageously, the composition of the invention is stable and the particles remain suspended in the composition for an extended time period, such as, for example greater than 6 months, more typically greater than one year at ambient temperature, as well as under accelerated aging conditions, such as, for example, greater than three months storage at 45°C.

Another advantageous feature of the compositions including a copolymer emulsion of the invention is that they exhibit a clear, transparent visual appearance even at acidic pH ranges.

So, even under acidic pH conditions, the copolymer emulsion of the present invention provides thickening properties and imparts a non-zero yield strength without imparting a visually turbid appearance to the composition, thus allowing formulation of visually clear, viscous compositions having a non-zero yield strength.

In a particular embodiment, the invention relates to compositions comprising a liquid medium and one or more copolymers of the present invention, in particular to personal care compositions further comprising one or more surfactants, with said compositions exhibiting clear, transparent visual appearance, for example exhibiting a light transmittance value at 600 nm of greater than 85%, preferably of greater than 88% and more preferably greater than 90%..

### Applications

The copolymer emulsion of the present invention is particularly useful in, for example, personal care applications, such as, for example, cosmetic formulations, personal care products, shampoos, body wash, hand soap, lotions, creams, conditioners, shaving products, facial washes, neutralizing shampoos, personal wipes, health care products, and skin treatments, and in home care or institutional or industrial care applications, such as, for example, liquid detergents, laundry detergents, hard surface cleansers, dish wash liquids, toilet bowl cleaners, as well as other applications, such as oil field, paper coating field, paints field, in the manufacture of aqueous suspensions of mineral materials or of formulations containing hydraulic binder, and agrochemical or chemical processes applications as for example rheology modifiers, film-formers, thickeners, emulsifiers, stabilizers, solubilizers, suspending agents, and pigment grinding additives.

According to a particular embodiment variant, the present invention is directed to a personal or topical health care composition that comprises a copolymer emulsion of the present invention.

Suitable personal care compositions, such as cosmetics, toiletries, health and beauty aids, cosmeceuticals) and topical health care compositions include without limitation, hair care products, such as shampoos (including combination shampoos, such as "two-in-one" conditioning shampoos), hair conditioners, post-shampoo rinses, hair colorants, setting, styling, and style maintenance agents including setting aids, such as gels and sprays, grooming aids, such as pomades, conditioners, perms, relaxers, hair smoothing products, and the like, skin care products (facial, body, hands, scalp and feet), such as creams, lotions, conditioners, and cleansing products, such as facial washes and body washes, anti-acne products, anti-aging products (exfoliant, keratolytic, anticellulite, antiwrinkle, and the like), skin protectants such as sunscreens, sunblock, barrier creams, oils, silicones, and the like, skin color products (whiteners, lighteners, sunless tanning accelerators, and the like), hair colorants (hair dyes, hair color rinses, highlighters, bleaches and the like), pigmented skin colorants (face and body makeups, foundation creams, mascara, rouge, lip products, and the like), bath and shower products (body cleansers, body wash, shower gel, liquid soap, soap bars, syndet bars, conditioning liquid bath oil, bubble bath, bath powders, and the like), nail care products (polishes, polish removers, strengtheners, lengtheners, hardeners, cuticle removers, softeners, and the like), oral care products, such as toothpastes and mouthcare products.

Compositions for personal care and topical health care can be in the form of, without being limited thereto, liquids, such as rinses, gels, sprays, emulsions, such as lotions and creams, shampoos, pomades, foams, ointments, tablets, sticks, such as lip care products, makeup, and suppositories, and like products, which are applied to skin and hair and remain in contact therewith until removed as by rinsing with water or washing with shampoo or soap. Gels can be soft, stiff, or squeezable. Emulsions can be oil-in-water, water-in-oil, or multiphase. Sprays can be non-pressurized aerosols delivered from manually pumped finger-actuated sprayers or can be pressurized aerosols. The copolymer of the present invention can be formulated in an aerosol composition, such as in a spray, mousse, or foam forming formulation, where a chemical or gaseous propellant is required. Physiologically and environmentally tolerable propellants, such as compressed gases, fluorinated hydrocarbons and liquid volatile hydrocarbons, and the amounts and suitable combinations to be used, are well known in the cosmetic and pharmaceutical art and literature.

According to another embodiment variant, the present invention is directed a home care composition or institutional or industrial cleaning composition, such as a liquid detergent, a laundry detergent, a hard surface cleanser, a dish wash liquid, or a toilet bowl cleaner, comprising water, one or more surfactants, and a copolymer emulsion of the present invention. Suitable home care composition or institutional or industrial cleaning compositions include surface cleansers for kitchen and bathroom counter tops, tiled surfaces, and utilities, including appliances employed or located therein, toilet cleaners, including toilet bowl rim gels, floor cleansers, wall cleansers, polishes, air freshener gels, detergents, treatments and cleansers for dishes and laundry, such as fabric softener, spot reducer, and fabric treatments

As indicated previously, the liquid composition of the present invention may comprise an amount of the polymer of the present invention that is effective to impart a yield strength of greater than 1 Pa to the composition and the composition further comprises suspended particles of one or more solid, liquid, or gas that is insoluble or are only partly soluble in the composition, such as, for example, abrasives, pigments, oil droplets, oil beads, liposomes, capsules, or gas bubbles.

The materials or compounds which require stabilization and/or suspension are generally partially soluble or insoluble in water. Such compounds include but are not limited to insoluble silicones, silicone gums and resins, volatile and nonvolatile silicone oils, natural and synthetic waxes and oils and fatty acids, opacifying or pearlescent materials, pharmaceutical and cosmeceutical active (such as vitamins, skin and hair nourishing or protecting compounds, skin lighteners etc.), particulates, pigments, exfoliating agents, anti-dandruff agents, botanical material extracts, fragrances. Other generally insoluble components suitable for use in the present compositions include clay, swellable clay, laponite, gas bubbles, liposomes, microsponges, cosmetic beads, capsules and flakes that can be included in a composition for aesthetic appearance or can function as micro- and macro-encapsulants for the delivery of benefit agents to the skin and hair.

The invention will now be described in further details using the following non-limiting examples.

### EXAMPLES

In the following examples, the aqueous surfactant formulations incorporating the different copolymer emulsions were prepared according to the following protocol. The amounts are indicated in percent by weight of active material (wt% active) relative to the total weight of the composition.

The copolymer emulsion, Sodium laureth-2EO sulfate ("SLES", as Rhodapex ES-2K) and Cocamidopropylbetaine ("CAPB", as Mirataine BET C-30) were added to most part of deionized water under moderate agitation.

A solution of sodium hydroxide (15%) was slowly added to the mixture, so as to reach a solution pH of about 6.5. Sodium chloride was added if necessary.

Then, formulation pH was adjusted to pH 4.7- 5.0 with a solution of citric acid (15%). 0.5 wt% sodium benzoate (preservative) was added under moderate agitation. Final pH was adjusted to [4.9 = 5.1] with a solution of sodium hydroxide (15%) or citric acid (15%) and the rest of deionized water available was added up to 100.

The formulations were centrifuged to remove any entrained air bubbles. The sample jars containing the centrifuged formulations were capped and held for 24 hours after which following characterizations are made.

### Characterizations

Each of the compositions was tested and evaluated for their clarity, suspending and thickening properties, pursuant to the testing procedure protocols described below.

### Yield Stress (also referred to as yield strength)

Flow curves and yield stress measurements are conducted on an advanced rheometer (TA instruments AR2000 Rheometer), using specific protocols: measurements carried out at 25°C using a 60-mm 2°-cone/plate geometry with a 53 µm-gap. After a 2-min stabilization step, a stepped flow is applied with a ramp shear rate from 10⁻³s⁻¹ to 1s⁻¹ (20 points per decade in 10 min). A continuous ramp is then applied from 1s⁻¹ to 1000s⁻¹ (20 points/ decade in 3 min).

The static yield stress is determined as the value of the applied shear stress beyond which the viscosity starts decreasing. It is usually recognized that suspension properties will increase when yield stress increases.

Yield stress values greater than or equal to 0.5, preferably greater than or equal 1 are suitable for the desired formulations, in order to obtain suitable suspension properties of pigments, particles, beads, capsules, gas bubbles etc.

### brookfield Viscosity

Brookfield Viscosity of the compositions was measured using a Brookfield Viscometer Model RV DVD-I or DVII + (Brookfield Engineering Laboratories, Inc.) at 10 revolutions per minute (RPM), with spindles 4 or 5. Measurements were conducted at temperature between 19 and 23°C.

Typical order of magnitude of Brookfield Viscosity (10 rpm) for the desired thickened formulations arc 2,500 - 40,000 mPa.s, preferably 2,500 - 25,000 mPa.s.

### Transmittance

The clarity was indicated by transmittance (%T) measurements at 600 nm in 2.5ml polystyrene cell, 10 x 10 mm, using a UV/VIS spectrometer Lambda Bio 40.

In the case of the following examples, it is considered that compositions having a transmittance of 88% or more, preferably more than to 90%, were substantially clear.

### Suspension

Suspension properties of the final formulations were assessed visually by checking the suspension of Polyethylene beads (Metapearls-1 STD Blue) homogeneously dispersed into the considered formulation (0.1 grams of beads in 50 grams of formulation), after storage for three months at 45°C.

### EXAMPLE 1

### Preparation of the copolymer emulsions

The ingredients used are summarized in TABLE 1 below. The polymers of Examples E1-E6, each contained:
- optionally first monomeric units derived from acrylic acid ("AA"),
- second monomeric units derived from methacrylic acid ("MA"),
- third monomeric units derived from ethyl acrylate ("EA"),
- optionally fourth monomeric units derived from sodium salt of acrylamide-2-methylpropane sulfonic acid,
- fifth monomeric units derived from a monomeric compound according to structure (IV') above, wherein R¹⁹ = methyl, i = 25, and j = 5 ("NOPOL polyether monomer"),
- sixth monomeric units derived from a mixture of (C₁₆-C₂₂)alkyl-polyethoxylated methacrylates having an average of 25 ethylene oxide units per molecule, according to structure (V), wherein R²⁵ is methyl, R²¹ is a mixture of linear C₁₆ alkyl, linear C₁₈ alkyl, and linear C₂₂ alkyl groups, p = 2, r = 25 s =0, and t = 1 ("(C₁₆C₂₂)alkyl-polyether monomer").

The NOPOL polyether monomer was introduced in the form of an aqueous solution ("NOPOL polyether monomer solution") that contained, based on 100 wt% active of the solution, about 50 wt% active of the NOPOL polyether monomer and about 25 wt% active MA.

The (C₁₆-C₂₂)alkyl-polyether monomer was introduced in the form of an aqueous solution ("(C₁₆-C₂₂)alkyl-polyether solution") that contained, based on 100 wt% active of the solution, about 50 wt% active of the (C₁₆-C₂₂)alkyl-polyether monomer and about 25 wt% active MA.

In a 1 L jacketed reactor, the water (300.20gr) and the surfactant ammonium laureth sulfate (Rhodapcx AB20 - ammonium salt of sulphated alcohol ethoxylate, 29wt% active material in water) (10.34 gr) were added. The reactor contents was heated to 78°C and purged with N₂. Once temperature was stable, 6.54g of sodium salt of 2-acrylamido-2-methylpropane sulfonic acid (51wt% in water), 0.449g of sodium persulfate dissolved in 5g of water and 0.05g of sodium metabisulfite dissolved in 5g of water were added. Then 25% of a mixture of 90.1g of ethyl acrylate, 39.8g of acrylic acid and 0.152g of trymetylolpropane diallyleter (TMPDE, 90% active material) were continuously added. After the addition of the 25% of the monomer mixture, the remaining 75% mixed with 12.09g of NOPOL polyether monomer solution as is and 25.06g of (C₁₆-C₂₂)alkyl-polyether solution as is were added. The latex was aged for 30 minutes at 78°C. 0.050g of sodium persulfate dissolved in 25g of water were semi continuously added. The redaction contents was cooled down and the reaction vessel emptied.

Copolymer emulsions referenced E2, E3, E4, E5 and E6 were synthesized using the same process with the different wt% monomer compositions indicated in Table 1 below.

The amounts of monomers for each of the synthetized copolymer emulsions are expressed as weight percent of active material (wt% active) based upon the total monomer active weight content.

The amount of surfactant for each of the synthetized copolymer emulsions are expressed as weight percent of active material (wt% active) based upon the total monomer active weight content

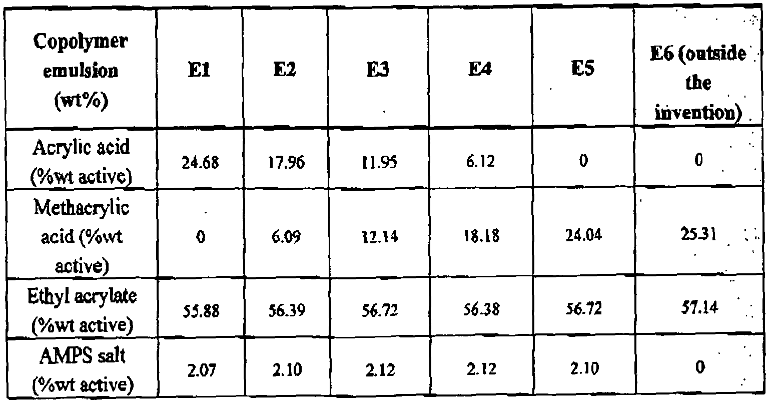

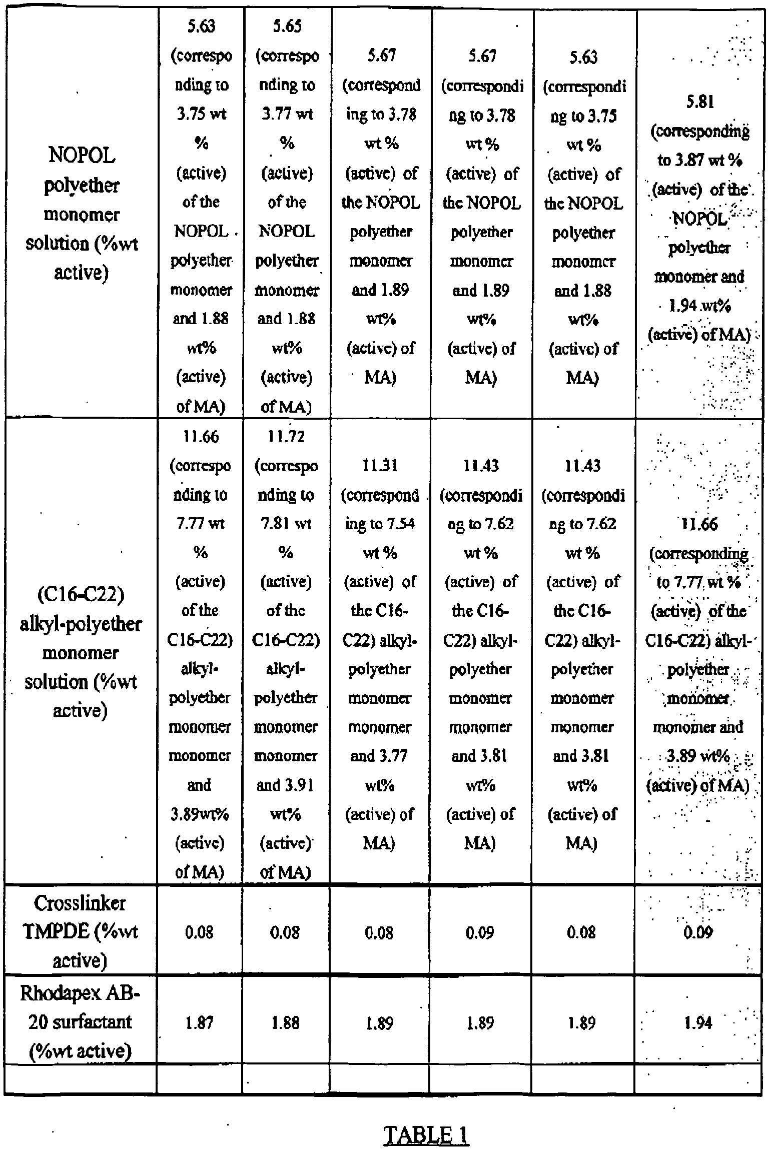

### Results

Aqueous surfactant formulations F1 to F6 were prepared with the synthesized copolymer emulsions E1 to E6 according to the above-mentioned protocol with the proportions: 2.5 wt% active copolymer emulsion in an aqueous composition containing 9.0 wt% active of SLES and 2.0wt% active CAPB. pH of the aqueous composition was adjusted to [4.9-5.1] after neutralisation at 6.5.

The aqueous surfactant formulations were then tested pursuant to the testing procedure protocols described above.

The results are recorded in Table 2 below.

**TABLE 2**

| **Formalations** | **F1** | **F2** | **F3** | **F4** | **F5** | **F6** |
|---|---|---|---|---|---|---|
| Copolymer emulsion used | E1 | E2 | E3 | E4 | E5 | E6 |
| Brookfield Viscosity between 2500 and 25000 mPa.s ? | Yes | Yes | Yes | Yes | Yes | Yes |
| | | | | | | |
| Transmittance (%) | 93.1 | 93.4 | 93.2 | 91.7 | 89.6 | 83.6 |
| Yield Stress (Pa) | 1.1 | 1.5 | 1.0 | 1.4 | 2.3 | 4.9 |
| | | | | | | |
| Suspension beads (3 months 45°C) | Yes | Yes | Yes | Yes | Yes | Yes |

All formulations have adequate viscosity and suspension properties. The copolymer emulsions of the invention E1 to E5 demonstrate better transmittance indicating better clarity compared to non-conform copolymer emulsion E6.

### EXAMPLE 2

### Preparation of the copolymer emulsions

The ingredients used are summarized in TABLE 3 below. The polymers of Examples E7-E11, each contained:
- optionally first monomeric units derived from acrylic acid ("AA"),
- second monomeric units derived from methacrylic acid ("MA"),
- third monomeric units derived from ethyl acrylate ("EA"),
- optionally fourth monomeric units derived from sodium salt of acrylamide-2-methylpropane sulfonic acid,
- fifth monomeric units derived from a monomeric compound according to structure (IV') above, wherein R¹⁹ = methyl, i = 25, and j = 5 ("NOPOL polyether monomer"),
- sixth monomeric units derived from a mixture of (C₁₆-C₂₂)alkyl-polyethoxylated methacrylates having an average of 25 ethylene oxide units per molecule, according to structure (V), wherein R²⁵ is methyl, R²¹ is a mixture of linear C₁₆ alkyl, linear C₁₈ alkyl, and linear C₂₂ alkyl groups, p = 2, r = 25 s =0, and t = 1 ("(C₁₆-C₂₁)alkyl-polyether monomer").

The NOPOL polyether monomer was introduced in the form of an aqueous solution ("NOPOL polyether monomer solution") that contained, based on 100 wt% active of the solution, about 50 wt% active of the NOPOL polyether monomer and about 25 wt% active MA.

The (C₁₆-C₂₂)alkyl-polyether monomer was introduced in the form of an aqueous solution ("(C₁₆-C₂₂)alkyl-polyether solution") that contained, based on 100 wt% active of the solution, about 50 wt% active of the (C₁₆-C₂₂)alkyl-polyether monomer and about 25 wt% active MA.

Copolymer emulsion referenced E7 was prepared according to the following process. In a 1L jacketed reactor, the water (300.20g) and 10.34g of the surfactant ammonium laureth sulfate (Rhodapex AB20 ammonium salt of sulphated alcohol ethoxylate, 29wt% active material in water) were added. The reactor contents was heated to 78°C and purged with N₂. Once temperature was stable, 6.0g of sodium salt of 2-acrylamido-2-methylpropane sulfonic acid (AMPS - 51 wt% in water), 0.449g of sodium persulfate dissolved in 5g of water and 0.05g of sodium metabisulfite dissolved in 5g of water were added. Then 25% of a mixture of 88.3g of ethyl acrylate, 3.0 g of acrylic acid and 35.4g of methacrylic acid were continuously added. 50% of this monomer mixture mixed with 8g of NOPOL polyether monomer solution as is and 16g of (C₁₆=C₂₂)alkyl-polyether solution as is were added. Then the last 25% of this monomer mixture with 4g of NOPOL polyether monomer solution as is, 8.1g of (C₁₆-C₂₂)alkyl-polyether solution as is, and 0.144g of Trimethylolpropane diallyl ether (TMPDE, 90% active material) were added. The reactor temperature was increased to 87°C in 30 minutes. Once temperature has been stable at 87°C, 0.23g of terbutyl hydroperoxyde TBHP (75% active) was added shot-wise, followed by a continuous addition of 0.16g of erythorbic acid dissolved in 10g of water (30 minutes). The latex was aged for 30 minutes at 87°C. After the 30 minute hold, 0.23g of terbutyl hydroperoxyde TBHP (75% active) was added shot-wise followed by a continuous addition of 0.16g of erythorbic acid dissolved in 10g of water. The reaction contents were cooled down and the reaction vessel emptied.

Copolymer emulsion referenced E8, E9 and E10 were synthesized using the same process with the different wt% monomer compositions indicated in Table 3 below. Copolymer emulsion referenced E11 were synthesized using the same process, but with sodium lauryl sulfate (Rhodapon LS-92RN) as a surfactant instead of Rhodapex AB20, with the wt% monomer compositions indicated in Table 3 below.

The amounts of monomers for each of the synthetized copolymer emulsions are expressed as weight percent of active material (wt% active) based upon the total monomer active weight content.

The amount of surfactant for each of the synthetized copolymer emulsions are expressed as weight percent of active material (wt% active) based upon the total monomer active weight content

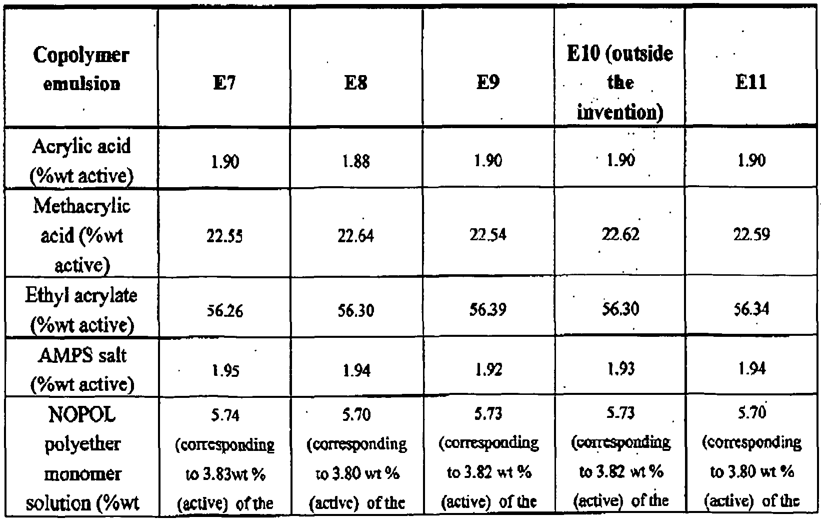

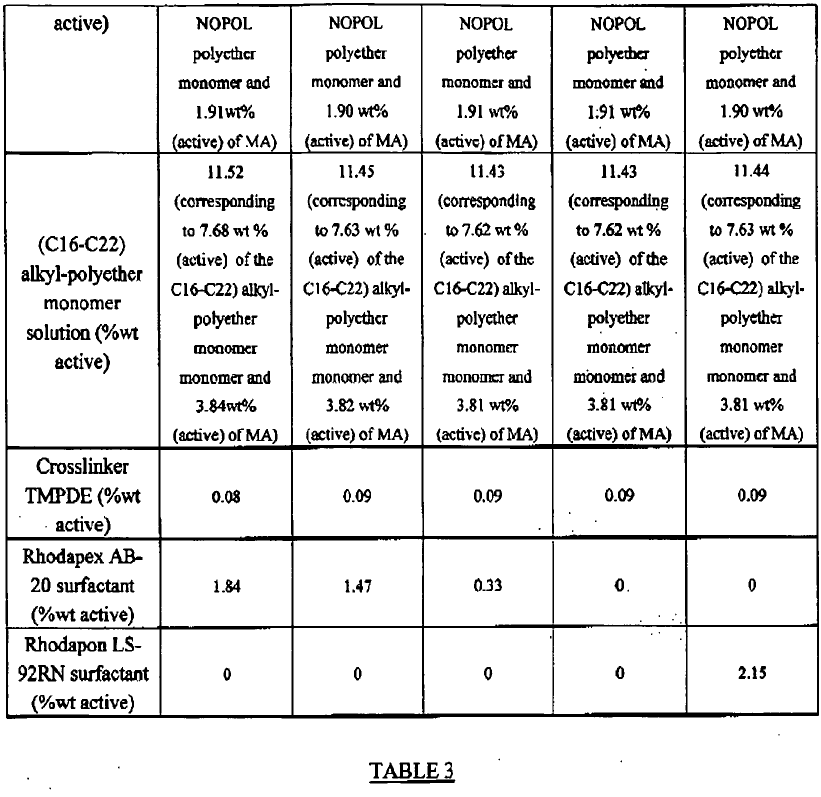

### Results

Aqueous surfactant formulations F7 to F11 were prepared with the synthetized copolymer emulsions E7 to E11 according to the above-mentioned protocol with the proportions: 2.5 wt% active copolymer emulsion in an aqueous composition containing 9.0 wt% active of SLES and 2.0wt% active CAPB. pH of the aqueous composition was adjusted to [4.9-5.1] after neutralisation at 6.5.

The aqueous surfactant formulations were then tested pursuant to the testing procedure protocols described above.

The results are recorded in Table 4 below.

**TABLE 4**

| **Formulations** | **F7** | **F8** | **F9** | **F10** | **F11** |
|---|---|---|---|---|---|
| Copolymer emulsion used | E7 | E8 | E9 | E10 | E11 |
| Brookfield Viscosity between 2500 and 25000 mPa.s ? | Yes | Yes | Yes | Yes | Yes |
| | | | | | |
| Transmittance (%) | 94.1 | 943 | 89.8 | 63.7 | 95.2 |
| Yield Stress (Pa) | 2.2 | 2.1 | 3.3 | 4.8 | 2.25 |
| | | | | | |
| Suspension beads (3 months 45°C) | Yes | Yes | Yes | Yes | Yes |

All formulations have adequate viscosity and suspension properties.

Formulations F7, F8, F9 and F11 with copolymer emulsions in accordance with the invention have a combination of superior clarity, viscosity and suspension properties.

However, the copolymer emulsion free of surfactant delivers a decreased transmittance. The formulation is turbid.

### EXAMPLE 3

### Comparison with benchmark rheology modifiers tin rinse-off formulations at different pH

The use of the copolymer emulsion E7 (prepared according to example 2) was compared with the use of benchmark rheology modifiers Carbopol Aqua SF-2 from Lubrizol and Eliclear 4U from Seppic, in aqueous formulations at different acidic pH.

Aqueous surfactant formulations were prepared according to the above-mentioned protocol with the proportions: 2.5 wt% active copolymer emulsion in an aqueous composition containing 9-0 wt% active of SLES and 2.0wt% active CAPB. pH of the aqueous composition was adjusted to 5.0; 4.5 or 4.0 after neutralisation at 6.5.

The aqueous surfactant formulations were then tested pursuant to the testing procedure protocols described above.

The results are recorded in Table 5 below.

**TABLE 5**

| **Formulations** | **F7** | **Comparative F12** | **Comparative F13** |
|---|---|---|---|
| Copolymer emulsion used | E7 | Carbopol Aqua SF-2 | Eliclear 4U |

| **pH adjustment at 5.0** | | | |
|---|---|---|---|
| Brookfield Viscosity between 2500 and 25000 mPa.s ? | Yes | Yes | Yes |
| Transmittance (%) | 94.1 | 96.6 | 87.4 |
| Yield Stress (Pa) | 2.2 | 0.8 | 4.5 |
| Suspension beads (3 months 45°C) | Yes | Yes (partial) | Yes |

| **pH adjustment at 4.5** | | | |
|---|---|---|---|
| Brookfield Viscosity between 2500 and 25000 mPa.s ? | Yes | Yes | Yes |
| Transmittance (%) | 94.6 | 95.1 | 87.1 |
| Yield Stress (Pa) | 2.2 | 1.6 | 3.9 |
| Suspension beads (3 months 45°C) | Yes | Yes | Yes |

| **pH adjustment at 4.0** | | | |
|---|---|---|---|
| Brookfield Viscosity between 2500 and 25000 mPa.s ? | Yes | Yes | Yes |
| Transmittance (%) | 94.4 | 96.2 | 86.7 |
| Yield Stress (Pa) | 2.3 | 2.0 | 4.6 |
| Suspension beads (3 months 45°C) | Yes | Yes | Yes |

At pH 5.0, 4.5 or 4.0, in the considered rinse-off formulations, Eliclear 4U show transmittance below 88%, which corresponds to hazy formulation.

At pH 5.0; 4.5 or 4.0, copolymer emulsion E7 and commercial Carbopol Aqua SF-2 display transmittance higher than 90%, which corresponds to clear formulations.

At pH 5.0; 4.5 or 4.0, copolymer emulsion E7 shows a higher yield stress than Carbopol Aqua SF-2, i.e. better suspension ability.

In conclusion, copolymer emulsion E7 of the invention has better combined yield value and clarity properties.

### EXAMPLE 4

Aqueous surfactant formulations F14 and F15 were prepared according to the above-mentioned protocol with the proportions indicated in table 6 below. pH of the aqueous formulations was adjusted to around 5 after neutralisation at 6.5.

**TABLE 6**

| **Formulations composition (wt% active content)** | **F14** | **F15** |
|---|---|---|
| E7 | 2.50 | 2.50 |
| SLES | 9.0 | 9.0 |
| CAPB | 2.0 | 2.0 |
| Sodium chloride | 0.0 | 1.01 |
| Sodium benzoate | 0.52 | 0.53 |

The formulations were then tested pursuant to the testing procedure protocols described above.

The results are recorded in Table 7 below.

**TABLE 7**

| **Characterization** | **F14** | **F15** |
|---|---|---|
| pH | 5.01 | 5.06 |
| Brookfield Viscosity (mPa.s) | 9880 | 13860 |
| Trsrismittance % | 94.1 | 92.2 |
| Yield Stress (Pa) | 2.2 | 2.9 |
| Suspension beads (3 months 45°C) | yes | yes |

Formulations containing active sodium chloride also show a high viscosity, a high transmittance superior to 90%, a high yield stress with high suspension ability.

This demonstrates that the copolymers of the invention are not overly sensitive to salt content, particularly at pH below 6. The formulations containing a copolymer of the invention does not typically undergo undesirable changes in response to relatively small changes in the amount of salts.

### EXAMPLE 5

Aqueous surfactant formulations F16 and F17 were prepared according to the above-mentioned protocol with the proportions indicated in table 8 below. pH of the aqueous formulations was adjusted to around 5 after neutralisation at 6.5.

**TABLE 8**

| **Formulation composition (wt% active content)** | **F14** | **F16** | **F17** |
|---|---|---|---|
| E7 | 2.50 | 2.52 | 2.50 |
| SLES | 9.0 | 7.80 | 6.62 |
| CAPB | 2.0 | 3.16 | 4.47 |
| Sodium chloride | 0.0 | 0.0 | 0.0 |
| Sodium benzoate | 0.52 | 0.51 | 0.53 |

The formulations were then tested pursuant to the testing procedure protocols described above.

The results are recorded in Table 9 below.

**TABLE 9**

| **Characterization** | **F14** | **F16** | **F17** |
|---|---|---|---|
| pH | 5.01 | 5.07 | 5.02 |
| Brookfield Viscosity (mPa.s) | 9880 | 12320 | 17560 |
| Transmittance % | 94.1 | 93.6 | 90.5 |
| Yield Stress (Pa) | 2.2 | 2.7 | 3.2 |
| Suspension beads (3 months 45°C) | yes | yes | yes |

All formulations show a high viscosity, a high transmittance superior to 90%, a high yield stress with high suspension ability,

This demonstrates that the formulations containing a copolymer of the invention do not typically undergo undesirable changes in response to changes in the surfactant system.

### EXAMPLE 6

### Liquid hand-soap formulation

The copolymer emulsion E7 (prepared according to example 2), sodium laureth-2EO sulfate ("SLES", as Rhodapex ES-2K) and sodium cocoamphoacetate ("CAMA", as Miranol Ultra C-32) were added to most part of deionized water under moderate agitation. A solution of sodium hydroxide (15%) was slowly added to the mixture, so as to reach a solution pH of about 6.5. Formulation pH was adjusted to pH 4.7-5.0 with a solution of citric acid (15%). 0.5 wt% sodium benzoate (preservative) was added under moderate agitation. Final pH was adjusted to 5.0 with a solution of sodium hydroxide (15%) or citric acid (15%) and the rest of deionized water available was added up to 100.

The composition of the hand-soap formulation is summarized in the following Table 10.

**TABLE 10**

| **Formulation composition** | **wt% active content** |
|---|---|
| E7 | 2.48 |
| SLES | 9.73 |
| CAMA | 2.28 |
| Sodium benzoate | 0.50 |

The formulation was then tested pursuant to the testing procedure protocols described above.

The results are recorded in Table 11 below.

**TABLE 11**

| **Characterization** | |
|---|---|
| pH | 5.0 |
| Brookfield Viscosity (mPa.s) | 13900 |
| Transmittance % | 93.4 |
| Yield Stress (Pa) | 2.7 |
| Suspension beads (3 months 45°C) | yes |

Liquid hand-soap formulation shows a high viscosity, a high transmittance superior to 90%, a high yield stress with high suspension ability.

### EXAMPLE 7

### Shampoo

Ammonium lauryl sulfate ("ALS", as Rhodapon ALSA B) and Cocamide monoethanolamine ("Cocamide MEA", as Mackamide CMA) were added to half of available amount of deionized water under moderate agitation. The mixture was heated at 60°C until homogeneous. Cocamidopropylbetaine ("CAPB", as Mirataine BET C-30) was added, followed by copolymer emulsion E7 under moderate agitation. A solution of sodium hydroxide (15%) was slowly added to the mixture, so as to reach a solution pH of about 6.5. Formulation pH was adjusted to pH 5.0- 5.3 with a solution of citric acid (15%). 0.5 wt% sodium benzoate (preservative) was added under moderate agitation. Final pH was adjusted to 5.3 with a solution of sodium hydroxide (15%) or citric acid (15%) and the rest of deionized water available was added up to 100.

The composition of the shampoo is summarized in the following Table 12.

**TABLE 12**

| **Formulation composition** | **wt% active content** |
|---|---|
| E7 | 2.06 |
| ALS | 10.51 |
| CAPB | 2.11 |
| CMEA | 1.00 |
| Sodium benzoate | 0.52 |

The formulation was then tested pursuant to the testing procedure protocols described above.

The results are recorded in Table 13 below.

**TABLE 13**

| **Characterization** | |
|---|---|
| pH | 5.3 |
| Brookfield Viscosity (mPa.s) | 13460 |
| Transmittance % | 96.0 |
| Yield Stress (Pa) | 1.2 |
| Suspension beads (3 months 45°C) | yes |

The shampoo formulation shows a high viscosity, a high transmittance superior to 90%, a high yield stress with high suspension ability.

### EXAMPLE 8

### Conditioning Shampoo

Copolymer emulsion E8 was added in deionized water, followed by addition of Ammonium lauryl sulfate ("ALS", as Rhodapon ALSA B) under moderate agitation. In a separate vessel, a solution of 6.67wt% active of Cocamide monoethanolamine ("Cocamide MEA", as Mackamide CMA) in deionized water was prepared, under heating at 60°C. Once homogeneous, the solution of Cocamide MEA was cooled down to ambient temperature, and then added to the mixture under moderate agitation. A solution of sodium hydroxide (15%) was slowly added to the mixture, so as to reach a solution pH of about 6.5. Cocamidopropylbetaine ("CAPB", as Mirataine BET C-30) was then added. Hydroxypropyl guar hydroxypropyltrimonium chloride ("HPguarHPTC", as Jaguar C-162) was added under high speed agitation (1000 RPM during 10minutes), followed by addition of 0.5 wt% sodium benzoate (preservative). Formulation pH was adjusted to pH 5.3 with a solution of citric acid (15%) and the rest of deionized water available was added up to 100.

The composition of the conditioning shampoo is summarized in the following Table 13.

**TABLE 13**

| **Formulation composition** | **wt% active content** |
|---|---|
| E8 | 2.0 |
| ALS | 12.7 |
| CAPB | 2.6 |
| CMEA | 1.0 |
| HPguarHPTC | 0.3 |
| Sodium benzoate | 0.5 |

The formulation was then tested pursuant to the testing procedure protocols described above.

The results are recorded in Table 14 below.

**TABLE 14**

| **Characterization** | |
|---|---|
| pH | 5.3 |
| Brookfield Viscosity (mPa.s) | 35560 |
| Transmittance % | 95.2 |
| Yield Stress (Pa) | 1.9 |
| Suspension beads (3 months 45°C) | yes |

Conditioning shampoo formulation shows a high viscosity, a high transmittance superior to 90%, a high yield stress with high suspension ability.

### EXAMPLE 9

### Shampoo and/or shower gel compositions

The copolymer E8 of the invention can be used in the following formulations.

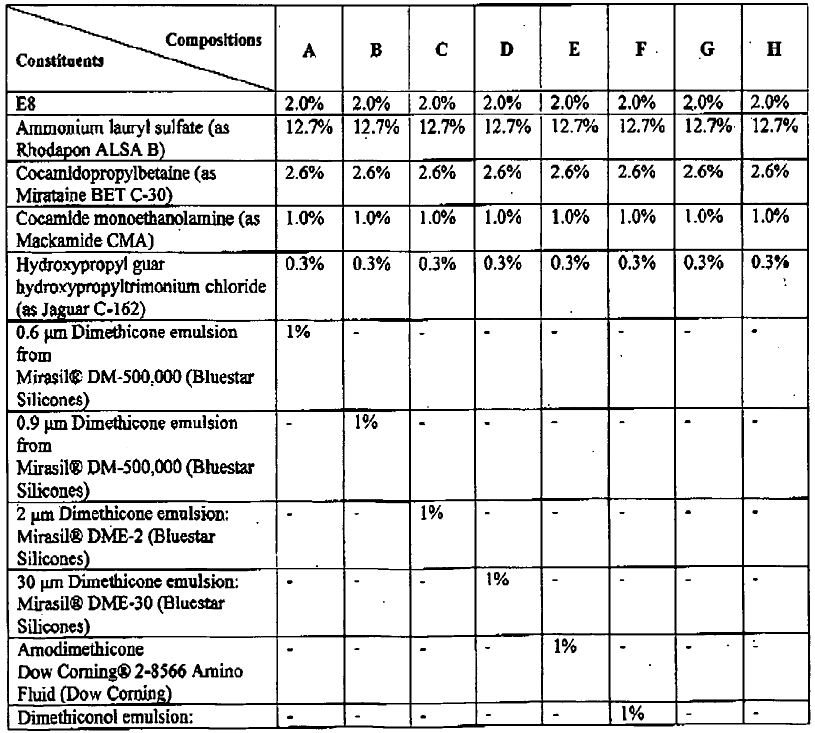

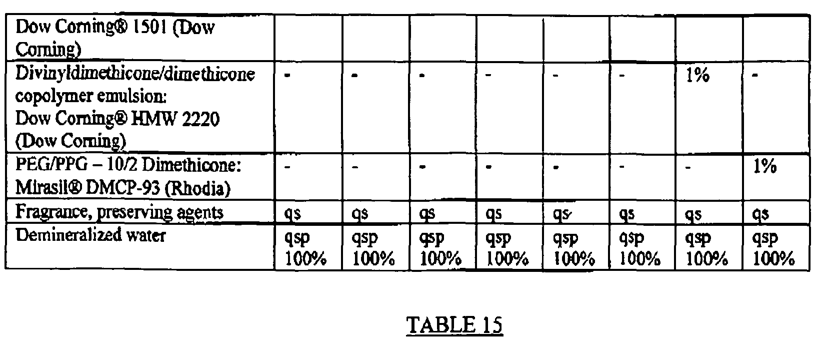

### EXAMPLE 10

### Shampoo and/or shower gel compositions

The copolymer E8 of the invention can be used in the following formulations.

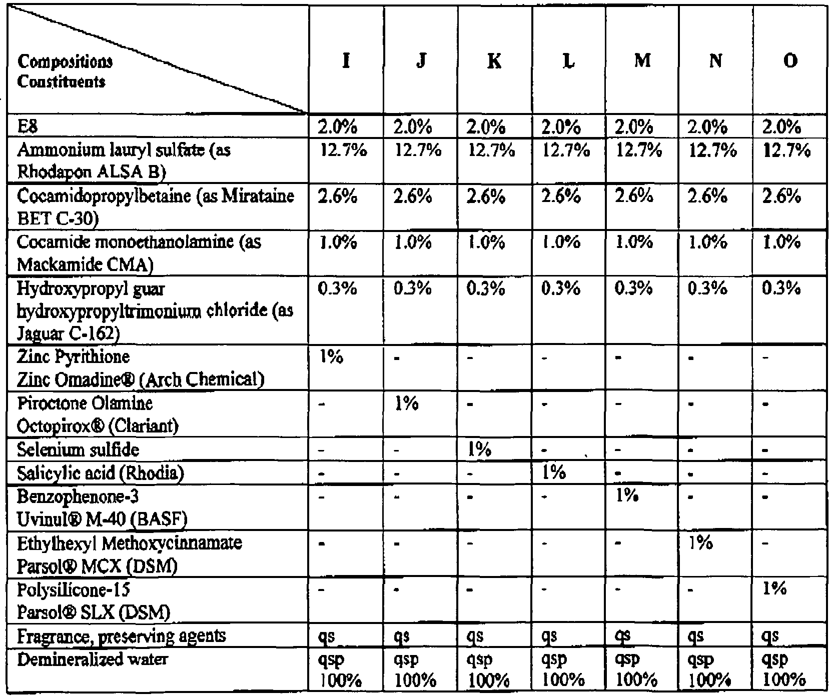

## Claims

1. A method for making a direct emulsion of a copolymer in water, **characterized in that** it comprises the polymerization of at least, expressed as a percentage by weight of each of the monomers based upon the total weight of the monomers:
a) 10 to 80% by weight of methacrylic acid and, optionally, of acrylic acid;
b) 15 to 80% by weight of at least one non-ionic vinyl monomer;
c) 0.05 to 9.5% by weight of 2-acrylamido-2-methylpropane sulfonic acid or a salt thereof;
d) 0.5 to 30% by weight of at least one monomer containing at least one hydrophobic group; and
e) 0.01 to 5% by weight of at least one crosslinking monomer;
said polymerization being carried out in the presence of at least one surfactant

2. The method according to claim 1, **characterized in that** it comprises the polymerization of at least 10.5 to 80% by weight of methacrylic acid and of acrylic acid.

3. The method according to claim 1 or 2, **characterized in that** the total of a), b), c), d) and e) weight contents is equal to 100%.

4. The method according to any one of the preceding claims, wherein the total weight amount of methacrylic acid and, optionally, of acrylic acid, for example the total weight amount of methacrylic acid and of acrylic acid, ranges from 20% to 50%, in particular from 25% to 40%, based upon the total weight of the monomers forming the copolymer.

5. The method according to any one of the preceding claims, wherein the non-ionic vinyl monomer is an ester of (meth)acrylic acid, for example chosen from Cₜ-C₁₈-alkyl esters of acrylic acid and/or of methacrylic acid, in particular chosen from ethyl acrylate, butyl acrylate and methyl methacrylate.

6. The method according to any one of the preceding claims, wherein the weight amount of non-ionic vinyl monomer(s) is greater than or equal to 30%, for example ranges from 30% to 80%, for example is greater than or equal to 40%, for example is greater than or equal to 50%, for example ranges from 50% to 75%, based upon the total weight of the monomers forming the copolymer.

7. The method according to any one of the preceding claims, wherein the weight amount of acrylamido-2-methylpropanesulfonic acid or a salt thereof ranges from 0.1% to 7%, in particular from 0.5% to 5% and more particularly from 1% to 3%, based upon the total weight of the monomers forming the copolymer.

8. The method according to any one of the preceding claims, wherein the monomer containing at least one hydrophobic group has general structure (III): 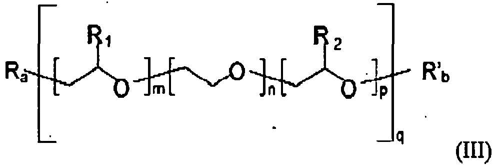 wherein:
- m, n, p and q are integers and m, n, p are inferior to 150, q is superior to 0 and at least one integer from m, n and p is non-zero,
- Ra has a polymerizable vinylic function;
- R₁ and R₂ are indentical or different and represent hydrogen atoms or alkyl groups,
- R'b is a hydrophobic group containing from 6 to at most 36 carbon atoms.

9. A direct emulsion of a copolymer in water, wherein the copolymer is polymerized from a monomer mixture comprising at least, expressed as a percentage by weight of each of the monomers based upon the total weight of monomers:
(a) 10 to 80% by weight of methacrylic acid and, optionally, of acrylic acid;
(b) 15 to 80% by weight of at least one non-ionic vinyl monomer;
(c) 0.05 to 9.5% by weight of 2-acrylamido-2-methylpropane sulfonic acid or a salt thereof;
(d) 0.5 to 30% by weight of at least one monomer containing at least one hydrophobic group; and
(e) 0.01 to 5% by weight of at least one crosslinking monomer;
wherein said emulsion contains at least one surfactant.

10. The emulsion according to claim 9, wherein a), b), c), d), e) monomers are as described in any one of claims 2 to 8.

11. A method for thickening an aqueous composition comprising an emulsion of a copolymer according to any one of claims 9 to 10, comprising adding to said aqueous composition a pH adjusting agent selected from an acidic material, an alkaline material and mixtures thereof.

12. A method according to the preceding claim, wherein said aqueous composition further includes at least a surfactant different from the surfactant contained in said emulsion of the copolymer, in particular selected from anionic, zwitterionic or amphoteric, cationic or non-ionic surfactants, and combinations thereof.

13. An aqueous composition comprising the copolymer emulsion as claimed in any one of claims 9 to 10.

14. The composition according to the preceding claim, said composition having a pH value equal to or lower than 6, in particular equal to or lower than 5.5, more particularly ranging from 2 to 5, in particular ranging from 3 to 5.

15. The composition according to any one of claims 13 to 14, wherein the composition comprises an amount of said copolymer effective to impart a yield strength of greater than 0 Pa to the composition, in particular greater than 0.5 Pa and more particularly greater than 1 Pa.

16. The composition according to any one of claims 13 to 15, wherein the composition comprises an amount of said copolymer effective to impart a yield strength of greater than 1 Pa to the composition and the composition further comprises suspended particles of one or more solid, liquid, or gas that are insoluble or are only partly soluble in the composition.

17. The composition according to any one of claims 13 to 16, wherein the composition exhibits a clear, transparent visual appearance.

## Patentansprüche

1. Verfahren zur Herstellung einer direkten Emulsion eines Copolymers in Wasser, **dadurch gekennzeichnet, dass** es die Polymerisation von mindestens, ausgedrückt als Gewichtsprozentanteil jedes der Monomere, bezogen auf das Gesamtgewicht der Monomere :
a) 10 bis 80 Gew.- % Methacrylsäure und gegebenenfalls Acrylsäure;
b) 15 bis 80 Gew.- % mindestens eines nichtionischen Vinylmonomers;
c) 0,05 bis 9,5 Gew.- % 2-Acrylamido-2-methylpropansulfonsäure oder eines Salzes davon;
d) 0,5 bis 30 Gew.- % mindestens eines Monomers mit mindestens einer hydrophoben Gruppe und
e) 0,01 bis 5 Gew.- % mindestens eines Vernetzungsmonomers
umfasst; wobei die Polymerisation in Gegenwart von mindestens einem Tensid durchgeführt wird.

2. Verfahren nach Anspruch 1, **dadurch gekennzeichnet, dass** es die Polymerisation von mindestens 10,5 bis 80 Gew.- % Methacrylsäure und Acrylsäure umfasst.

3. Verfahren nach Anspruch 1 oder 2, **dadurch gekennzeichnet, dass** die Summe der Gewichtsgehalte von a), b), c), d) und e) gleich 100 % ist.

4. Verfahren nach einem der vorhergehenden Ansprüche, bei dem die gesamte Gewichtsmenge von Methacrylsäure und gegebenenfalls Acrylsäure, beispielsweise die gesamte Gewichtsmenge von Methacrylsäure und Acrylsäure, im Bereich von 20 % bis 50 %, insbesondere von 25 % bis 40 %, bezogen auf das Gesamtgewicht der das Copolymer bildenden Monomere, liegt.

5. Verfahren nach einem der vorhergehenden Ansprüche, bei dem es sich bei dem nichtionischen Vinylmonomer um einen Ester von (Meth)acrylsäure, beispielsweise ausgewählt aus C₁-C₁₈-Alkylestern von Acrylsäure und/oder Methacrylsäure, insbesondere ausgewählt aus Ethylacrylat, Butylacrylat und Methylmethacrylat, handelt.

6. Verfahren nach einem der vorhergehenden Ansprüche, bei dem die Gewichtsmenge von nichtionischem Vinylmonomer bzw. nichtionischen Vinylmonomeren größer gleich 30 % ist, beispielsweise im Bereich von 30 % bis 80 % liegt, beispielsweise größer gleich 40 % ist, beispielsweise größer gleich 50 % ist, beispielsweise im Bereich von 50 % bis 75 % liegt, bezogen auf das Gesamtgewicht der das Copolymer bildenden Monomere.

7. Verfahren nach einem der vorhergehenden Ansprüche, bei dem die Gewichtsmenge von Acrylamido-2-methylpropansulfonsäure oder einem Salz davon im Bereich von 0,1 % bis 7 %, insbesondere von 0,5 % bis 5 % und spezieller von 1 % bis 3 %, bezogen auf das Gesamtgewicht der das Copolymer bildenden Monomere, liegt.

8. Verfahren nach einem der vorhergehenden Ansprüche, bei dem das Monomer mit mindestens einer hydrophoben Gruppe die allgemeine Struktur (III) aufweist: 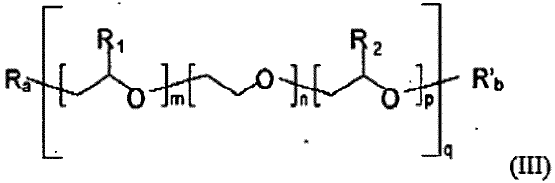 wobei:
- m, n, p und q für ganze Zahlen stehen und m, n, p kleiner als 150 sind, q größer als 0 ist und mindestens eine ganze Zahl von m, n und p nicht null ist,
- Ra eine polymerisierbare Vinylfunktion aufweist;
- R₁ und R₂ gleich oder verschieden sind und für Wasserstoffatome oder Alkylgruppen stehen;
- R'b für eine hydrophobe Gruppe mit 6 bis höchstens 36 Kohlenstoffatomen steht.

9. Direkte Emulsion eines Copolymers in Wasser, wobei das Copolymer aus einer Monomerenmischung polymerisiert wird, die mindestens, ausgedrückt als Gewichtsprozentanteil jedes der Monomere, bezogen auf das Gesamtgewicht der Monomere :
(a) 10 bis 80 Gew.- % Methacrylsäure und gegebenenfalls Acrylsäure;
(b) 15 bis 80 Gew.- % mindestens eines nichtionischen Vinylmonomers;
(c) 0,05 bis 9,5 Gew.- % 2-Acrylamido-2-methylpropansulfonsäure oder eines Salzes davon;
(d) 0,5 bis 30 Gew.- % mindestens eines Monomers mit mindestens einer hydrophoben Gruppe und
(e) 0,01 bis 5 Gew.- % mindestens eines Vernetzungsmonomers
umfasst; wobei die Emulsion mindestens ein Tensid enthält.

10. Emulsion nach Anspruch 9, wobei die Monomere a), b), c), d), e) wie in einem der Ansprüche 2 bis 8 beschrieben sind.

11. Verfahren zum Verdicken einer wässrigen Zusammensetzung, die eine Emulsion eines Copolymers nach einem der Ansprüche 9 bis 10 umfasst, bei dem man die wässrige Zusammensetzung mit einem aus einer sauren Substanz, einer alkalischen Substanz und Mischungen davon ausgewählten Mittel zur Einstellung des pH-Werts versetzt.

12. Verfahren nach dem vorhergehenden Anspruch, bei dem die wässrige Zusammensetzung ferner mindestens ein Tensid, das von dem in der Emulsion des Copolymers enthaltenen Tensid verschieden ist, insbesondere ausgewählt aus anionischen, zwitterionischen oder amphoteren, kationischen oder nichtionischen Tensiden und Kombinationen davon, umfasst.

13. Wässrige Zusammensetzung, umfassend die Copolymeremulsion gemäß einem der Ansprüche 9 bis 10.

14. Zusammensetzung nach dem vorhergehenden Anspruch, wobei die Zusammensetzung einen pH-Wert kleiner gleich 6, insbesondere kleiner gleich 5,5, spezieller im Bereich von 2 bis 5, insbesondere im Bereich von 3 bis 5, aufweist.

15. Zusammensetzung nach einem der Ansprüche 13 bis 14, wobei die Zusammensetzung eine Menge des Copolymers umfasst, die der Zusammensetzung eine Fließgrenze von mehr als 0 Pa, insbesondere mehr als 0,5 Pa und spezieller mehr als 1 Pa verleiht.

16. Zusammensetzung nach einem der Ansprüche 13 bis 15, wobei die Zusammensetzung eine Menge des Copolymers umfasst, die der Zusammensetzung eine Fließgrenze von mehr als 1 Pa verleiht, und die Zusammensetzung ferner suspendierte Teilchen aus einem oder mehreren Feststoffen, Flüssigkeiten oder Gasen, die in der Zusammensetzung unlöslich oder nur teilweise löslich sind, umfasst.

17. Zusammensetzung nach einem der Ansprüche 13 bis 16, wobei die Zusammensetzung ein klares, transparentes visuelles Erscheinungsbild zeigt.

## Revendications

1. Procédé de fabrication d'une émulsion directe d'un copolymère dans de l'eau, **caractérisé en ce qu'**il comprend la polymérisation d'au moins, exprimés en pourcentage pondéral de chacun des monomères relativement au poids total des monomères :
a) 10 à 80 % en poids d'acide méthacrylique et, optionnellement, d'acide acrylique ;
b) 15 à 80 % en poids d'au moins un monomère vinylique non ionique ;
c) 0,05 à 9,5 % en poids d'acide 2-acrylamido-2-méthylpropanesulfonique ou d'un sel de celui-ci ;
d) 0,5 à 30 % en poids d'au moins un monomère contenant au moins un groupe hydrophobe ; et
e) 0,01 à 5 % en poids d'au moins un monomère de réticulation ;
ladite polymérisation étant effectuée en présence d'au moins un tensioactif.

2. Procédé selon la revendication 1, **caractérisé en ce qu'**il comprend la polymérisation d'au moins 10,5 à 80 % en poids d'acide méthacrylique et d'acide acrylique.

3. Procédé selon la revendication 1 ou 2, **caractérisé en ce que** le total des quantités en poids de a), b), c), d) et e) est égal à 100 %.

4. Procédé selon l'une quelconque des revendications précédentes, dans lequel la quantité totale en poids d'acide méthacrylique et, optionnellement, d'acide acrylique, par exemple la quantité totale en poids d'acide méthacrylique et d'acide acrylique, est de 20 % à 50 %, en particulier de 25 % à 40 %, relativement au poids total des monomères formant le copolymère.

5. Procédé selon l'une quelconque des revendications précédentes, dans lequel le monomère vinylique non ionique est un ester d'acide (méth)acrylique, sélectionné par exemple parmi des alkylesters C₁-C₁₈ d'acide acrylique et/ou d'acide méthacrylique, sélectionné en particulier parmi l'acrylate d'éthyle, l'acrylate de butyle et le méthacrylate de méthyle.

6. Procédé selon l'une quelconque des revendications précédentes, dans lequel la quantité en poids de monomère(s) vinylique(s) non ionique(s) est supérieure ou égale à 30 %, est par exemple de 30 % à 80 %, est par exemple supérieure ou égale à 40 %, est par exemple supérieure ou égale à 50 %, est par exemple de 50 % à 75 %, relativement au poids total des monomères formant le copolymère.

7. Procédé selon l'une quelconque des revendications précédentes, dans lequel la quantité en poids d'acide acrylamido-2-méthylpropanesulfonique ou d'un sel de celui-ci est de 0,1 % à 7 %, en particulier de 0,5 % à 5 %, et plus particulièrement de 1 % à 3 %, relativement au poids total des monomères formant le copolymère.

8. Procédé selon l'une quelconque des revendications précédentes, dans lequel le monomère contenant au moins un groupe hydrophobe a la structure générale (III) : 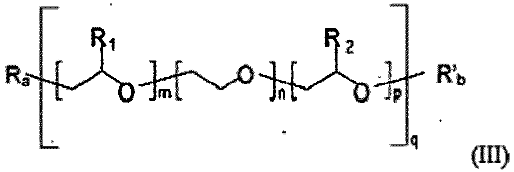 dans laquelle :
- m, n, p et q sont des nombres entiers et m, n, p sont inférieurs à 150, q est supérieur à 0 et au moins un nombre entier parmi m, n et p est différent de zéro,
- Ra a une fonction vinylique polymérisable ;
- R₁ et R₂ sont identiques ou différents et représentent des atomes d'hydrogène ou des groupes alkyle,
- R'b est un groupe hydrophobe contenant de 6 à un maximum de 36 atomes de carbone.

9. Émulsion directe d'un copolymère dans de l'eau, dans laquelle le copolymère est polymérisé à partir d'un mélange de monomères comprenant au moins, exprimés en pourcentage pondéral de chacun des monomères relativement au poids total des monomères :
(a) 10 à 80 % en poids d'acide méthacrylique et, optionnellement, d'acide acrylique ;
(b) 15 à 80 % en poids d'au moins un monomère vinylique non ionique ;
(c) 0,05 à 9,5 % en poids d'acide 2-acrylamido-2-méthylpropanesulfonique ou d'un sel de celui-ci ;
(d) 0,5 à 30 % en poids d'au moins un monomère contenant au moins un groupe hydrophobe ; et
(e) 0,01 à 5 % en poids d'au moins un monomère de réticulation ;
ladite émulsion contenant au moins un tensioactif.

10. Émulsion selon la revendication 9, dans laquelle les monomères a), b), c), d), e) sont tels que décrits dans l'une quelconque des revendications 2 à 8.

11. Procédé d'épaississement d'une composition aqueuse comprenant une émulsion d'un copolymère selon l'une quelconque des revendications 9 ou 10, comprenant l'addition à ladite composition aqueuse d'un agent d'ajustement du pH sélectionné parmi une matière acide, une matière alcaline, et des mélanges de celles-ci.

12. Procédé selon la revendication précédente, dans lequel ladite composition aqueuse comprend en outre au moins un tensioactif différent du tensioactif contenu dans ladite émulsion du copolymère, sélectionné en particulier parmi des tensioactifs anioniques, zwittérioniques ou amphotères, cationiques ou non ioniques, et des combinaisons de ceux-ci.

13. Composition aqueuse comprenant l'émulsion de copolymère selon l'une quelconque des revendications 9 ou 10.

14. Composition selon la revendication précédente, ladite composition ayant une valeur de pH égale ou inférieure à 6, en particulier égale ou inférieure à 5,5, plus particulièrement de 2 à 5, en particulier de 3 à 5.

15. Composition selon l'une quelconque des revendications 13 ou 14, la composition comprenant une quantité dudit copolymère efficace pour donner une limite élastique supérieure à 0 Pa à la composition, en particulier supérieure à 0,5 Pa, et plus particulièrement supérieure à 1 Pa.

16. Composition selon l'une quelconque des revendications 13 à 15, la composition comprenant une quantité dudit copolymère efficace pour donner une limite élastique supérieure à 1 Pa à la composition, et la composition comprenant en outre des particules en suspension d'un ou plusieurs solides, liquides, ou gaz, qui sont insolubles ou sont seulement partiellement solubles dans la composition.

17. Composition selon l'une quelconque des revendications 13 à 16, la composition présentant une apparence visuelle incolore et transparente.
